# EUROPÄISCHE PATENTSCHRIFT

(11) **EP 2 392 591 B1**
(45) Veröffentlichungstag und Bekanntmachung des Hinweises auf die Patenterteilung: **21.02.2018**
(21) Anmeldenummer: 11005620.7
(22) Anmeldetag: 11.06.2003
(51) Int. Cl.: C07K 14/415, C12N 15/11, C12N 15/63, A61K 38/16, A61K 39/36, A61K 48/00

(54) **DNA-Sequenz und rekombinante Herstellung des Graspollen-Allergens Phl p 4**
DNA sequence and recombinant creation of grass pollen allergen Phl p 4
Séquence ADN et fabrication recombinante de l'allergène du pollen de graminée Phl p4

(30) Priorität: 25.06.2002 EP 02013953
(43) Veröffentlichungstag der Anmeldung: 07.12.2011
(62) Teilanmeldung aus: 03740216.1
(73) Patentinhaber: Merck Patent GmbH, 64293 Darmstadt (DE)
(72) Erfinder: Nandy, Andreas, Dr., 22175 Hamburg (DE); Fiebig, Helmut, Prof., Dr., 21493 Schwarzenbek (DE); Suck, Roland, Dr., 22143 Hamburg (DE); Cromwell, Oliver, Dr., 23701 Suesel-Fassenberg (DE); Petersen, Arnd, Dr., 23795 Bad Segeberg (DE); Becker, Wolf-Meinhard, Dr., 23975 Moezen (DE)

(56) Entgegenhaltungen:
- SUCK R ET AL: "COMPLEMENTARY DNA CLONING AND EXPRESSION OF A NEWLY RECOGNIZED HIGHMOLECULAR MASS ALLERGEN PHL P 13 FROM TIMOTHY GRASS POLLEN (PHLEUM PRATENSE)", CLINICAL AND EXPERIMENTAL ALLERGY, BLACKWELL SCIENTIFIC PUBLICATIONS, LONDON, GB, Bd. 30, Nr. 3, März 2000 (2000-03), Seiten 324-332, XP000953168, ISSN: 0954-7894
- SUCK R ET AL: "The high molecular mass allergen fraction of timothy grass pollen (Phleum pratense) between 50-60 kDa is comprised of two major allergens: Ph1 p 4 and Ph1 p 13", CLINICAL AND EXPERIMENTAL ALLERGY, Bd. 30, Nr. 10, Oktober 2000 (2000-10), Seiten 1395-1402, XP002260344, ISSN: 0954-7894
- FISHER S ET AL: "Characterization of Phl p4, a major timothy grass (Phleum pratense) pollen allergen", JOURNAL OF ALLERGY AND CLINICAL IMMUNOLOGY, MOSBY - YEARLY BOOK, INC, US, Bd. 98, Nr. 1, Juli 1996 (1996-07), Seiten 189-198, XP000953216, ISSN: 0091-6749
- FAHLBUSCH B ET AL: "Detection and quantification of group 4 allergens in grass pollen extracts using monoclonal antibodies", CLINICAL AND EXPERIMENTAL ALLERGY, Bd. 28, Nr. 7, Juli 1998 (1998-07), Seiten 799-807, XP002260345, ISSN: 0954-7894
- STUMVOLL SABINE ET AL: "Purification, structural and immunological characterization of a timothy grass (Phleum pratense) pollen allergen, Phl p 4, with cross-reactive potential.", BIOLOGICAL CHEMISTRY, Bd. 383, Nr. 9, September 2002 (2002-09), Seiten 1383-1396, XP002260346, ISSN: 1431-6730
- NANDY A ET AL: "Primary structure, recombinant expression, and molecular characterization of Phl p 4, a major allergen of timothy grass (Phleum pratense)", BIOCHEMICAL AND BIOPHYSICAL RESEARCH COMMUNICATIONS, ACADEMIC PRESS INC. ORLANDO, FL, US, Bd. 337, Nr. 2, 18. November 2005 (2005-11-18), Seiten 563-570, XP027218637, ISSN: 0006-291X, DOI: 10.1016/J.BBRC.2005.09.087 [gefunden am 2005-10-11]
- ANDERSSON ET AL: "Characteristics and immunobiology of grass pollen allergens", INTERNATIONAL ARCHIVES OF ALLERGY AND IMMUNOLOGY, KARGER AG, CH, Bd. 130, 1. Januar 2003 (2003-01-01), Seiten 87-107, XP009137010, ISSN: 1018-2438, DOI: 10.1159/000069013
- BIOLOGICAL ABSTRACTS, vol. -, no. -, 7 June 2003 (2003-06-07), Philadelphia, PA, US, abstract no.: 726, A NANDY, S BUCHHOP, R SUCK, A PETERSEN, O CROMWELL, H FIEBIG: "Unveiling the secrets of the primary structure of Phl p 4: Molecular cloning of the major pollen allergen from Timothy Grass (Phleum pratense)", page -,

### SEQUENCE LISTING

<110> Merck Patent GmbH
<120> DNA-Sequenz und rekombinante Herstellung des Graspollen-Allergens Phl p 4
<130> P 02/101 EP DIV1
<150> EP 02 013953.1
   <151> 2002-06-25
<150> EP 03 740216.1
   <151> 2003-06-11
<160> 52
<170> PatentIn version 3.5
<210> 1
   <211> 1503
   <212> DNA
   <213> Phleum pratense
<220>
   <221> artificial_DNA_sequence
   <222> (1)..(69)
   <223> DNA sequence derived from sequenced protein
<220>
   <221> CDS
   <222> (1)..(1503)
<220>
   <221> native_DNA_sequence
   <222> (70)..(1503)
<400> 1
   tac ttc ccg ccg ccg gct gct aaa gaa gac ttc ctg ggt tgc ctg gtt      48
   Tyr Phe Pro Pro Pro Ala Ala Lys Glu Asp Phe Leu Gly Cys Leu Val
   1               5                   10                  15
   aaa gaa atc ccg ccg cgt ctg ttg tac gcg aaa tcg tcg ccg gcg tat      96
   Lys Glu Ile Pro Pro Arg Leu Leu Tyr Ala Lys Ser Ser Pro Ala Tyr
               20                  25                  30
   ccc tca gtc ctg ggg cag acc atc cgg aac tcg cgg tgg tcg tcg ccg     144
   Pro Ser Val Leu Gly Gln Thr Ile Arg Asn Ser Arg Trp Ser Ser Pro
           35                  40                  45
   gac aac gtg aag ccg atc tac atc gtc acc ccc acc aac gcc tcc cac     192
   Asp Asn Val Lys Pro Ile Tyr Ile Val Thr Pro Thr Asn Ala Ser His
       50                  55                  60
   atc cag tcc gcc gtg gtg tgc ggc cgc cgg cac ggt gtc cgc atc cgc     240
   Ile Gln Ser Ala Val Val Cys Gly Arg Arg His Gly Val Arg Ile Arg
   65                  70                  75                  80
   gtg cgc agc ggc ggg cac gac tac gag ggc ctc tcg tac cgg tcc ctg     288
   Val Arg Ser Gly Gly His Asp Tyr Glu Gly Leu Ser Tyr Arg Ser Leu
                   85                  90                  95
   cag ccc gag gag ttc gcc gtc gtc gac ctt agc aag atg cgg gcc gtg     336
   Gln Pro Glu Glu Phe Ala Val Val Asp Leu Ser Lys Met Arg Ala Val
               100                 105                 110
   tgg gtg gac ggg aag gcc cgc acg gcg tgg gtc gac tcc ggc gcg cag     384
   Trp Val Asp Gly Lys Ala Arg Thr Ala Trp Val Asp Ser Gly Ala Gln
           115                 120                 125
   ctc ggc gag ctc tac tac gcc atc cac aag gcg agt aca gtg ctg gcg       432
   Leu Gly Glu Leu Tyr Tyr Ala Ile His Lys Ala Ser Thr Val Leu Ala
       130                 135                 140
   ttc ccg gcc ggc gtg tgc ccg acc atc ggc gtg ggc ggc aac ttc gcg       480
   Phe Pro Ala Gly Val Cys Pro Thr Ile Gly Val Gly Gly Asn Phe Ala
   145                 150                 155                 160
   ggc ggc ggc ttc ggc atg ctg ctg cgc aag tac ggc atc gcg gcc gag       528
   Gly Gly Gly Phe Gly Met Leu Leu Arg Lys Tyr Gly Ile Ala Ala Glu
                   165                 170                 175
   aac gtc atc gac gtg aag ctc gtc gac gcc aac ggc acg ctg cac gac       576
   Asn Val Ile Asp Val Lys Leu Val Asp Ala Asn Gly Thr Leu His Asp
               180                 185                 190
   aag aag tcc atg ggc gac gac cat ttc tgg gcc gtc agg ggc ggc ggg       624
   Lys Lys Ser Met Gly Asp Asp His Phe Trp Ala Val Arg Gly Gly Gly
           195                 200                 205
   ggc gag agc ttc ggc atc gtg gtc gcg tgg aag gtg agg ctc ctg ccg       672
   Gly Glu Ser Phe Gly Ile Val Val Ala Trp Lys Val Arg Leu Leu Pro
       210                 215                 220
   gtg ccg ccc acg gtg acc gtg ttc aag atc ccc aag aag gcg agc gag       720
   Val Pro Pro Thr Val Thr Val Phe Lys Ile Pro Lys Lys Ala Ser Glu
   225                 230                 235                 240
   ggc gcc gtg gac atc atc aac agg tgg cag gtg gtc gcg ccg cag ctc       768
   Gly Ala Val Asp Ile Ile Asn Arg Trp Gln Val Val Ala Pro Gln Leu
                   245                 250                 255
   ccc gac gac ctc atg atc cgc gtc atc gcg cag ggc ccc acg gcc acg       816
   Pro Asp Asp Leu Met Ile Arg Val Ile Ala Gln Gly Pro Thr Ala Thr
               260                 265                 270
   ttc gag gcc atg tac ctg ggc acc tgc caa acc ctg acg ccg atg atg       864
   Phe Glu Ala Met Tyr Leu Gly Thr Cys Gln Thr Leu Thr Pro Met Met
           275                 280                 285
   agc agc aag ttc ccg gag ctc ggc atg aac gcc tcg cac tgc aac gag       912
   Ser Ser Lys Phe Pro Glu Leu Gly Met Asn Ala Ser His Cys Asn Glu
       290                 295                 300
   atg tcg tgg atc cag tcc atc ccc ttc gtc cac ctc ggc cac agg gac       960
   Met Ser Trp Ile Gln Ser Ile Pro Phe Val His Leu Gly His Arg Asp
   305                 310                 315                 320
   aac atc gag gac gac ctc ctc aac cgg aac aac acc ttc aag ccc ttc      1008
   Asn Ile Glu Asp Asp Leu Leu Asn Arg Asn Asn Thr Phe Lys Pro Phe
                   325                 330                 335
   gcc gaa tac aag tcg gac tac gtc tac gag ccg ttc ccc aag agg gtg      1056
   Ala Glu Tyr Lys Ser Asp Tyr Val Tyr Glu Pro Phe Pro Lys Arg Val
               340                 345                 350
   tgg gag cag atc ttc agc acc tgg ctc ctg aag ccc ggc gcg ggg atc      1104
   Trp Glu Gln Ile Phe Ser Thr Trp Leu Leu Lys Pro Gly Ala Gly Ile
           355                 360                 365
   atg atc ttc gac ccc tac ggc gcc acc atc agc gcc acc ccg gag tgg      1152
   Met Ile Phe Asp Pro Tyr Gly Ala Thr Ile Ser Ala Thr Pro Glu Trp
       370                 375                 380
   gcg acg ccg ttc cct cac cgc aag ggc gtc ctc ttc aac atc cag tac      1200
   Ala Thr Pro Phe Pro His Arg Lys Gly Val Leu Phe Asn Ile Gln Tyr
   385                 390                 395                 400
   gtc aac tac tgg ttc gcc ccg gga gcc ggc gcg gcg cca ttg tcg tgg     1248
   Val Asn Tyr Trp Phe Ala Pro Gly Ala Gly Ala Ala Pro Leu Ser Trp
                   405                 410                 415
   agc aag gag atc tac aac tac atg gag cca tac gtg agc aag aac ccc     1296
   Ser Lys Glu Ile Tyr Asn Tyr Met Glu Pro Tyr Val Ser Lys Asn Pro
               420                 425                 430
   agg cag gcc tac gcc aac tac agg gac atc gac ctc ggg agg aac gag     1344
   Arg Gln Ala Tyr Ala Asn Tyr Arg Asp Ile Asp Leu Gly Arg Asn Glu
           435                 440                 445
   gtg gtg aac gac gtc tcc acc ttc agc agc ggt ttg gtg tgg ggc cag     1392
   Val Val Asn Asp Val Ser Thr Phe Ser Ser Gly Leu Val Trp Gly Gln
       450                 455                 460
   aaa tac ttc aag ggc aat ttc cag agg ctc gcc atc acc aag ggc aag     1440
   Lys Tyr Phe Lys Gly Asn Phe Gln Arg Leu Ala Ile Thr Lys Gly Lys
   465                 470                 475                 480
   gtg gat ccc acc gac tac ttc agg aac gag cag agc atc ccg ccg ctc     1488
   Val Asp Pro Thr Asp Tyr Phe Arg Asn Glu Gln Ser Ile Pro Pro Leu
                   485                 490                 495
   atc aaa aag tac tga                                                 1503
   Ile Lys Lys Tyr
               500
<210> 2
   <211> 500
   <212> PRT
   <213> Phleum pratense
<400> 2
   Tyr Phe Pro Pro Pro Ala Ala Lys Glu Asp Phe Leu Gly Cys Leu Val
   1 5 10 15
   Lys Glu Ile Pro Pro Arg Leu Leu Tyr Ala Lys Ser Ser Pro Ala Tyr
   20 25 30
   Pro Ser Val Leu Gly Gln Thr Ile Arg Asn Ser Arg Trp Ser Ser Pro
   35 40 45
   Asp Asn Val Lys Pro Ile Tyr Ile Val Thr Pro Thr Asn Ala Ser His
   50 55 60
   Ile Gln Ser Ala Val Val Cys Gly Arg Arg His Gly Val Arg Ile Arg
   65 70 75 80
   Val Arg Ser Gly Gly His Asp Tyr Glu Gly Leu Ser Tyr Arg Ser Leu
   85 90 95
   Gln Pro Glu Glu Phe Ala Val Val Asp Leu Ser Lys Met Arg Ala Val
   100 105 110
   Trp Val Asp Gly Lys Ala Arg Thr Ala Trp Val Asp Ser Gly Ala Gln
   115 120 125
   Leu Gly Glu Leu Tyr Tyr Ala Ile His Lys Ala Ser Thr Val Leu Ala
   130 135 140
   Phe Pro Ala Gly Val Cys Pro Thr Ile Gly Val Gly Gly Asn Phe Ala
   145 150 155 160
   Gly Gly Gly Phe Gly Met Leu Leu Arg Lys Tyr Gly Ile Ala Ala Glu
   165 170 175
   Asn Val Ile Asp Val Lys Leu Val Asp Ala Asn Gly Thr Leu His Asp
   180 185 190
   Lys Lys Ser Met Gly Asp Asp His Phe Trp Ala Val Arg Gly Gly Gly
   195 200 205
   Gly Glu Ser Phe Gly Ile Val Val Ala Trp Lys Val Arg Leu Leu Pro
   210 215 220
   Val Pro Pro Thr Val Thr Val Phe Lys Ile Pro Lys Lys Ala Ser Glu
   225 230 235 240
   Gly Ala Val Asp Ile Ile Asn Arg Trp Gln Val Val Ala Pro Gln Leu
   245 250 255
   Pro Asp Asp Leu Met Ile Arg Val Ile Ala Gln Gly Pro Thr Ala Thr
   260 265 270
   Phe Glu Ala Met Tyr Leu Gly Thr Cys Gln Thr Leu Thr Pro Met Met
   275 280 285
   Ser Ser Lys Phe Pro Glu Leu Gly Met Asn Ala Ser His Cys Asn Glu
   290 295 300
   Met Ser Trp Ile Gln Ser Ile Pro Phe Val His Leu Gly His Arg Asp
   305 310 315 320
   Asn Ile Glu Asp Asp Leu Leu Asn Arg Asn Asn Thr Phe Lys Pro Phe
   325 330 335
   Ala Glu Tyr Lys Ser Asp Tyr Val Tyr Glu Pro Phe Pro Lys Arg Val
   340 345 350
   Trp Glu Gln Ile Phe Ser Thr Trp Leu Leu Lys Pro Gly Ala Gly Ile
   355 360 365
   Met Ile Phe Asp Pro Tyr Gly Ala Thr Ile Ser Ala Thr Pro Glu Trp
   370 375 380
   Ala Thr Pro Phe Pro His Arg Lys Gly Val Leu Phe Asn Ile Gln Tyr
   385                 390                 395                 400
   Val Asn Tyr Trp Phe Ala Pro Gly Ala Gly Ala Ala Pro Leu Ser Trp
                   405                 410                 415
   Ser Lys Glu Ile Tyr Asn Tyr Met Glu Pro Tyr Val Ser Lys Asn Pro
               420                 425                 430
   Arg Gln Ala Tyr Ala Asn Tyr Arg Asp Ile Asp Leu Gly Arg Asn Glu
           435                 440                 445
   Val Val Asn Asp Val Ser Thr Phe Ser Ser Gly Leu Val Trp Gly Gln
       450                 455                 460
   Lys Tyr Phe Lys Gly Asn Phe Gln Arg Leu Ala Ile Thr Lys Gly Lys
   465                 470                 475                 480
   Val Asp Pro Thr Asp Tyr Phe Arg Asn Glu Gln Ser Ile Pro Pro Leu
                   485                 490                 495
   Ile Lys Lys Tyr
               500
<210> 3
   <211> 1503
   <212> DNA
   <213> Phleum pratense
<220>
   <221> artificial_DNA_sequence
   <222> (1)..(69)
   <223> DNA sequence derived from sequenced protein
<220>
   <221> CDS
   <222> (1)..(1503)
<220>
   <221> native_DNA_sequence
   <222> (70)..(1503)
<400> 3
   tac ttc ccg ccg ccg gct gct aaa gaa gac ttc ctg ggt tgc ctg gtt        48
   Tyr Phe Pro Pro Pro Ala Ala Lys Glu Asp Phe Leu Gly Cys Leu Val
   1               5                   10                  15
   aaa gaa atc ccg ccg cgt ctg ttg tac gcg aaa tcg tcg ccg gcg tat        96
   Lys Glu Ile Pro Pro Arg Leu Leu Tyr Ala Lys Ser Ser Pro Ala Tyr
               20                  25                  30
   ccc tca gtc ctg ggg cag acc atc cgg aac tcg cgg tgg tcg tcg ccg       144
   Pro Ser Val Leu Gly Gln Thr Ile Arg Asn Ser Arg Trp Ser Ser Pro
           35                  40                  45
   gac aac gtg aag ccg atc tac atc gtc acc ccc acc aac gcc tcc cac       192
   Asp Asn Val Lys Pro Ile Tyr Ile Val Thr Pro Thr Asn Ala Ser His
       50                  55                  60
   atc cag tcc gcc gtg gtg tgc ggc cgc cgg cac ggt gtc cgc atc cgc       240
   Ile Gln Ser Ala Val Val Cys Gly Arg Arg His Gly Val Arg Ile Arg
   65                  70                  75                  80
   gtg cgc agc ggc ggg cac gac tac gag ggc ctc tcg tac cgg tcc ctg      288
   Val Arg Ser Gly Gly His Asp Tyr Glu Gly Leu Ser Tyr Arg Ser Leu
                   85                  90                  95
   cag ccc gag gag ttc gcc gtc gtc gac ctt agc aag atg cgg gcc gtg      336
   Gln Pro Glu Glu Phe Ala Val Val Asp Leu Ser Lys Met Arg Ala Val
               100                 105                 110
   tgg gtg gac ggg aag gcc cgc acg gcg tgg gtc gac tcc ggc gcg cag      384
   Trp Val Asp Gly Lys Ala Arg Thr Ala Trp Val Asp Ser Gly Ala Gln
           115                 120                 125
   ctc ggc gag ctc tac tac gcc atc cac aag gcg agt cca gtg ctg gcg      432
   Leu Gly Glu Leu Tyr Tyr Ala Ile His Lys Ala Ser Pro Val Leu Ala
       130                 135                 140
   ttc ccg gcc ggc gtg tgc ccg acc atc ggc gtg ggc ggc aac ttc gcg      480
   Phe Pro Ala Gly Val Cys Pro Thr Ile Gly Val Gly Gly Asn Phe Ala
   145                 150                 155                 160
   ggc ggc ggc ttc ggc atg ctg ctg cgc aag tac ggc atc gcg gcc gag      528
   Gly Gly Gly Phe Gly Met Leu Leu Arg Lys Tyr Gly Ile Ala Ala Glu
                   165                 170                 175
   aac gtc atc gac gtg aag ctc gtc gac gcc aac ggc acg ctg cac gac      576
   Asn Val Ile Asp Val Lys Leu Val Asp Ala Asn Gly Thr Leu His Asp
               180                 185                 190
   aag aag tcc atg ggc gac gac cat ttc tgg gcc gtc agg ggc ggc ggg      624
   Lys Lys Ser Met Gly Asp Asp His Phe Trp Ala Val Arg Gly Gly Gly
           195                 200                 205
   ggc gag agc ttc ggc atc gtg gtc gcg tgg aag gtg agg ctc ctg ccg      672
   Gly Glu Ser Phe Gly Ile Val Val Ala Trp Lys Val Arg Leu Leu Pro
       210                 215                 220
   gtg ccg ccc acg gtg acc gtg ttc aag atc ccc aag aag gcg agc gag      720
   Val Pro Pro Thr Val Thr Val Phe Lys Ile Pro Lys Lys Ala Ser Glu
   225                 230                 235                 240
   ggc gcc gtg gac atc atc aac agg tgg cag gtg gtc gcg ccg cag ctc      768
   Gly Ala Val Asp Ile Ile Asn Arg Trp Gln Val Val Ala Pro Gln Leu
                   245                 250                 255
   ccc gac gac ctc atg atc cgc gtc atc gcg cag ggc ccc acg gcc acg      816
   Pro Asp Asp Leu Met Ile Arg Val Ile Ala Gln Gly Pro Thr Ala Thr
               260                 265                 270
   ttc gag gcc atg tac ctg ggc acc tgc caa acc ctg acg ccg atg atg      864
   Phe Glu Ala Met Tyr Leu Gly Thr Cys Gln Thr Leu Thr Pro Met Met
           275                 280                 285
   agc agc aag ttc ccc gag ctc ggc atg aac gcc tcg cac tgc aac gag      912
   Ser Ser Lys Phe Pro Glu Leu Gly Met Asn Ala Ser His Cys Asn Glu
       290                 295                 300
   atg tcg tgg atc cag tcc atc ccc ttc gtc cac ctc ggc cac agg gac      960
   Met Ser Trp Ile Gln Ser Ile Pro Phe Val His Leu Gly His Arg Asp
   305                 310                 315                 320
   aac atc gag gac gac ctc ctc aac cgg aac aac acc ttc aag ccc ttc     1008
   Asn Ile Glu Asp Asp Leu Leu Asn Arg Asn Asn Thr Phe Lys Pro Phe
                   325                 330                 335
   gcc gaa tac aag tcg gac tac gtc tac gag ccg ttc ccc aag gaa gtg     1056
   Ala Glu Tyr Lys Ser Asp Tyr Val Tyr Glu Pro Phe Pro Lys Glu Val
               340                 345                 350
   tgg gag cag atc ttc agc acc tgg ctc ctg aag ccc ggc gcg ggg atc      1104
   Trp Glu Gln Ile Phe Ser Thr Trp Leu Leu Lys Pro Gly Ala Gly Ile
           355                 360                 365
   atg atc ttc gac ccc tac ggc gcc acc atc agc gcc acc ccg gag tgg      1152
   Met Ile Phe Asp Pro Tyr Gly Ala Thr Ile Ser Ala Thr Pro Glu Trp
       370                 375                 380
   gcg acg ccg ttc cct cac cgc aag ggc gtc ctc ttc aac atc cag tac      1200
   Ala Thr Pro Phe Pro His Arg Lys Gly Val Leu Phe Asn Ile Gln Tyr
   385                 390                 395                 400
   gtc aac tac tgg ttc gcc ccg gga gcc ggc gcg gcg cca ttg tcg tgg      1248
   Val Asn Tyr Trp Phe Ala Pro Gly Ala Gly Ala Ala Pro Leu Ser Trp
                   405                 410                 415
   agc aag gag atc tac aac tac atg gag cca tac gtg agc aag aac ccc      1296
   Ser Lys Glu Ile Tyr Asn Tyr Met Glu Pro Tyr Val Ser Lys Asn Pro
               420                 425                 430
   agg cag gcc tac gcc aac tac agg gac atc gac ctc ggg agg aac gag      1344
   Arg Gln Ala Tyr Ala Asn Tyr Arg Asp Ile Asp Leu Gly Arg Asn Glu
           435                 440                 445
   gtg gtg aac gac gtc tcc acc ttc agc agc ggt ttg gtg tgg ggc cag      1392
   Val Val Asn Asp Val Ser Thr Phe Ser Ser Gly Leu Val Trp Gly Gln
       450                 455                 460
   aaa tac ttc aag ggc aat ttc cag agg ctc gcc atc acc aag ggc aag      1440
   Lys Tyr Phe Lys Gly Asn Phe Gln Arg Leu Ala Ile Thr Lys Gly Lys
   465                 470                 475                 480
   gtg gat ccc acc gac tac ttc agg aac gag cag agc atc ccg ccg ctc      1488
   Val Asp Pro Thr Asp Tyr Phe Arg Asn Glu Gln Ser Ile Pro Pro Leu
                   485                 490                 495
   atc aaa aag tac tga                                                  1503
   Ile Lys Lys Tyr
               500
<210> 4
   <211> 500
   <212> PRT
   <213> Phleum pratense
<400> 4
   Tyr Phe Pro Pro Pro Ala Ala Lys Glu Asp Phe Leu Gly Cys Leu Val
   1               5                   10                  15
   Lys Glu Ile Pro Pro Arg Leu Leu Tyr Ala Lys Ser Ser Pro Ala Tyr
               20                  25                  30
   Pro Ser Val Leu Gly Gln Thr Ile Arg Asn Ser Arg Trp Ser Ser Pro
           35                  40                  45
   Asp Asn Val Lys Pro Ile Tyr Ile Val Thr Pro Thr Asn Ala Ser His
       50                  55                  60
   Ile Gln Ser Ala Val Val Cys Gly Arg Arg His Gly Val Arg Ile Arg
   65 70 75 80
   Val Arg Ser Gly Gly His Asp Tyr Glu Gly Leu Ser Tyr Arg Ser Leu
   85 90 95
   Gln Pro Glu Glu Phe Ala Val Val Asp Leu Ser Lys Met Arg Ala Val
   100 105 110
   Trp Val Asp Gly Lys Ala Arg Thr Ala Trp Val Asp Ser Gly Ala Gln
   115 120 125
   Leu Gly Glu Leu Tyr Tyr Ala Ile His Lys Ala Ser Pro Val Leu Ala
   130 135 140
   Phe Pro Ala Gly Val Cys Pro Thr Ile Gly Val Gly Gly Asn Phe Ala
   145 150 155 160
   Gly Gly Gly Phe Gly Met Leu Leu Arg Lys Tyr Gly Ile Ala Ala Glu
   165 170 175
   Asn Val Ile Asp Val Lys Leu Val Asp Ala Asn Gly Thr Leu His Asp
   180 185 190
   Lys Lys Ser Met Gly Asp Asp His Phe Trp Ala Val Arg Gly Gly Gly
   195 200 205
   Gly Glu Ser Phe Gly Ile Val Val Ala Trp Lys Val Arg Leu Leu Pro
   210 215 220
   Val Pro Pro Thr Val Thr Val Phe Lys Ile Pro Lys Lys Ala Ser Glu
   225 230 235 240
   Gly Ala Val Asp Ile Ile Asn Arg Trp Gln Val Val Ala Pro Gln Leu
   245 250 255
   Pro Asp Asp Leu Met Ile Arg Val Ile Ala Gln Gly Pro Thr Ala Thr
   260 265 270
   Phe Glu Ala Met Tyr Leu Gly Thr Cys Gln Thr Leu Thr Pro Met Met
   275 280 285
   Ser Ser Lys Phe Pro Glu Leu Gly Met Asn Ala Ser His Cys Asn Glu
   290 295 300
   Met Ser Trp Ile Gln Ser Ile Pro Phe Val His Leu Gly His Arg Asp
   305 310 315 320
   Asn Ile Glu Asp Asp Leu Leu Asn Arg Asn Asn Thr Phe Lys Pro Phe
   325 330 335
   Ala Glu Tyr Lys Ser Asp Tyr Val Tyr Glu Pro Phe Pro Lys Glu Val
   340 345 350
   Trp Glu Gln Ile Phe Ser Thr Trp Leu Leu Lys Pro Gly Ala Gly Ile
   355 360 365
   Met Ile Phe Asp Pro Tyr Gly Ala Thr Ile Ser Ala Thr Pro Glu Trp
   370 375 380
   Ala Thr Pro Phe Pro His Arg Lys Gly Val Leu Phe Asn Ile Gln Tyr
   385 390 395 400
   Val Asn Tyr Trp Phe Ala Pro Gly Ala Gly Ala Ala Pro Leu Ser Trp
   405 410 415
   Ser Lys Glu Ile Tyr Asn Tyr Met Glu Pro Tyr Val Ser Lys Asn Pro
   420 425 430
   Arg Gln Ala Tyr Ala Asn Tyr Arg Asp Ile Asp Leu Gly Arg Asn Glu
   435 440 445
   Val Val Asn Asp Val Ser Thr Phe Ser Ser Gly Leu Val Trp Gly Gln
   450 455 460
   Lys Tyr Phe Lys Gly Asn Phe Gln Arg Leu Ala Ile Thr Lys Gly Lys
   465 470 475 480
   Val Asp Pro Thr Asp Tyr Phe Arg Asn Glu Gln Ser Ile Pro Pro Leu
   485 490 495
   Ile Lys Lys Tyr
   500
<210> 5
   <211> 1503
   <212> DNA
   <213> Phleum pratense
<220>
   <221> artificial_DNA_sequence
   <222> (1)..(69)
   <223> DNA sequence derived from sequenced protein
<220>
   <221> CDS
   <222> (1)..(1503)
<220>
   <221> native_DNA_sequence
   <222> (70)..(1503)
<400> 5
   tac ttc ccg ccg ccg gct gct aaa gaa gac ttc ctg ggt tgc ctg gtt      48
   Tyr Phe Pro Pro Pro Ala Ala Lys Glu Asp Phe Leu Gly Cys Leu Val
   1               5                   10                  15
   aaa gaa atc ccg ccg cgt ctg ttg tac gcg aaa tcg tcg ccg gcg tat      96
   Lys Glu Ile Pro Pro Arg Leu Leu Tyr Ala Lys Ser Ser Pro Ala Tyr
               20                  25                  30
   ccc tca gtc ctg ggg cag acc atc cgg aac tcg agg tgg tcg tcg ccg      144
   Pro Ser Val Leu Gly Gln Thr Ile Arg Asn Ser Arg Trp Ser Ser Pro
           35                  40                  45
   gac aac gtg aag ccg ctc tac atc atc acc ccc acc aac gtc tcc cac      192
   Asp Asn Val Lys Pro Leu Tyr Ile Ile Thr Pro Thr Asn Val Ser His
       50                  55                  60
   atc cag tcc gcc gtg gtg tgc ggc cgc cgc cac agc gtc cgc atc cgc      240
   Ile Gln Ser Ala Val Val Cys Gly Arg Arg His Ser Val Arg Ile Arg
   65                  70                  75                  80
   gtg cgc agc ggc ggg cac gac tac gag ggc ctc tcg tac cgg tct ttg      288
   Val Arg Ser Gly Gly His Asp Tyr Glu Gly Leu Ser Tyr Arg Ser Leu
                   85                  90                  95
   cag ccc gag acg ttc gcc gtc gtc gac ctc aac aag atg cgg gcg gtg      336
   Gln Pro Glu Thr Phe Ala Val Val Asp Leu Asn Lys Met Arg Ala Val
               100                 105                 110
   tgg gtg gac ggc aag gcc cgc acg gcg tgg gtg gac tcc ggc gcg cag      384
   Trp Val Asp Gly Lys Ala Arg Thr Ala Trp Val Asp Ser Gly Ala Gln
           115                 120                 125
   ctc ggc gag ctc tac tac gcc atc tat aag gcg agc ccc acg ctg gcg      432
   Leu Gly Glu Leu Tyr Tyr Ala Ile Tyr Lys Ala Ser Pro Thr Leu Ala
       130                 135                 140
   ttc ccg gcc ggc gtg tgc ccg acg atc gga gtg ggc ggc aac ttc gcg      480
   Phe Pro Ala Gly Val Cys Pro Thr Ile Gly Val Gly Gly Asn Phe Ala
   145                 150                 155                 160
   ggc ggc ggc ttc ggc atg ctg ctg cgc aag tac ggc atc gcc gcg gag      528
   Gly Gly Gly Phe Gly Met Leu Leu Arg Lys Tyr Gly Ile Ala Ala Glu
                   165                 170                 175
   aac gtc atc gac gtg aag ctc gtc gac gcc aac ggc aag ctg cac gac      576
   Asn Val Ile Asp Val Lys Leu Val Asp Ala Asn Gly Lys Leu His Asp
               180                 185                 190
   aag aag tcc atg ggc gac gac cat ttc tgg gcc gtc agg ggc ggc ggg      624
   Lys Lys Ser Met Gly Asp Asp His Phe Trp Ala Val Arg Gly Gly Gly
           195                 200                 205
   ggc gag agc ttc ggc atc gtg gtc gcg tgg cag gtg aag ctc ctg ccg      672
   Gly Glu Ser Phe Gly Ile Val Val Ala Trp Gln Val Lys Leu Leu Pro
       210                 215                 220
   gtg ccg ccc acc gtg aca ata ttc aag atc tcc aag aca gtg agc gag      720
   Val Pro Pro Thr Val Thr Ile Phe Lys Ile Ser Lys Thr Val Ser Glu
   225                 230                 235                 240
   ggc gcc gtg gac atc atc aac aag tgg caa gtg gtc gcg ccg cag ctt      768
   Gly Ala Val Asp Ile Ile Asn Lys Trp Gln Val Val Ala Pro Gln Leu
                   245                 250                 255
   ccc gcc gac ctc atg atc cgc atc atc gcg cag ggg ccc aag gcc acg      816
   Pro Ala Asp Leu Met Ile Arg Ile Ile Ala Gln Gly Pro Lys Ala Thr
               260                 265                 270
   ttc gag gcc atg tac ctc ggc acc tgc aaa acc ctg acg ccg ttg atg      864
   Phe Glu Ala Met Tyr Leu Gly Thr Cys Lys Thr Leu Thr Pro Leu Met
           275                 280                 285
   agc agc aag ttc ccg gag ctc ggc atg aac ccc tcc cac tgc aac gag      912
   Ser Ser Lys Phe Pro Glu Leu Gly Met Asn Pro Ser His Cys Asn Glu
       290                 295                 300
   atg tca tgg atc cag tcc atc ccc ttc gtc cac ctc ggc cac agg gac       960
   Met Ser Trp Ile Gln Ser Ile Pro Phe Val His Leu Gly His Arg Asp
   305                 310                 315                 320
   gcc ctc gag gac gac ctc ctc aac cgg aac aac tcc ttc aag ccc ttc      1008
   Ala Leu Glu Asp Asp Leu Leu Asn Arg Asn Asn Ser Phe Lys Pro Phe
                   325                 330                 335
   gcc gaa tac aag tcc gac tac gtc tac cag ccc ttc ccc aag acc gtc      1056
   Ala Glu Tyr Lys Ser Asp Tyr Val Tyr Gln Pro Phe Pro Lys Thr Val
               340                 345                 350
   tgg gag cag atc ctc aac acc tgg ctc gtc aag ccc ggc gcc ggg atc      1104
   Trp Glu Gln Ile Leu Asn Thr Trp Leu Val Lys Pro Gly Ala Gly Ile
           355                 360                 365
   atg atc ttc gac ccc tac ggc gcc acc atc agc gcc acc ccg gag tcc      1152
   Met Ile Phe Asp Pro Tyr Gly Ala Thr Ile Ser Ala Thr Pro Glu Ser
       370                 375                 380
   gcc acg ccc ttc cct cac cgc aag ggc gtc ctc ttc aac atc cag tac      1200
   Ala Thr Pro Phe Pro His Arg Lys Gly Val Leu Phe Asn Ile Gln Tyr
   385                 390                 395                 400
   gtc aac tac tgg ttc gcc ccg gga gcc gcc gcc gcg ccc ctc tcg tgg      1248
   Val Asn Tyr Trp Phe Ala Pro Gly Ala Ala Ala Ala Pro Leu Ser Trp
                   405                 410                 415
   agc aag gac atc tac aac tac atg gag ccc tac gtg agc aag aac ccc      1296
   Ser Lys Asp Ile Tyr Asn Tyr Met Glu Pro Tyr Val Ser Lys Asn Pro
               420                 425                 430
   agg cag gcg tac gca aac tac agg gac atc gac ctc ggc agg aac gag      1344
   Arg Gln Ala Tyr Ala Asn Tyr Arg Asp Ile Asp Leu Gly Arg Asn Glu
           435                 440                 445
   gtg gtc aac gac gtc tcc acc tac gcc agc ggc aag gtc tgg ggc cag      1392
   Val Val Asn Asp Val Ser Thr Tyr Ala Ser Gly Lys Val Trp Gly Gln
       450                 455                 460
   aaa tac ttc aag ggc aac ttc gag agg ctc gcc att acc aag ggc aag      1440
   Lys Tyr Phe Lys Gly Asn Phe Glu Arg Leu Ala Ile Thr Lys Gly Lys
   465                 470                 475                 480
   gtc gat cct acc gac tac ttc agg aac gag cag agc atc ccg ccg ctc      1488
   Val Asp Pro Thr Asp Tyr Phe Arg Asn Glu Gln Ser Ile Pro Pro Leu
                   485                 490                 495
   atc aaa aag tac tga                                                  1503
   Ile Lys Lys Tyr
               500
<210> 6
   <211> 500
   <212> PRT
   <213> Phleum pratense
<400> 6
   Tyr Phe Pro Pro Pro Ala Ala Lys Glu Asp Phe Leu Gly Cys Leu Val
   1               5                   10                  15
   Lys Glu Ile Pro Pro Arg Leu Leu Tyr Ala Lys Ser Ser Pro Ala Tyr
   20 25 30
   Pro Ser Val Leu Gly Gln Thr Ile Arg Asn Ser Arg Trp Ser Ser Pro
   35 40 45
   Asp Asn Val Lys Pro Leu Tyr Ile Ile Thr Pro Thr Asn Val Ser His
   50 55 60
   Ile Gln Ser Ala Val Val Cys Gly Arg Arg His Ser Val Arg Ile Arg
   65 70 75 80
   Val Arg Ser Gly Gly His Asp Tyr Glu Gly Leu Ser Tyr Arg Ser Leu
   85 90 95
   Gln Pro Glu Thr Phe Ala Val Val Asp Leu Asn Lys Met Arg Ala Val
   100 105 110
   Trp Val Asp Gly Lys Ala Arg Thr Ala Trp Val Asp Ser Gly Ala Gln
   115 120 125
   Leu Gly Glu Leu Tyr Tyr Ala Ile Tyr Lys Ala Ser Pro Thr Leu Ala
   130 135 140
   Phe Pro Ala Gly Val Cys Pro Thr Ile Gly Val Gly Gly Asn Phe Ala
   145 150 155 160
   Gly Gly Gly Phe Gly Met Leu Leu Arg Lys Tyr Gly Ile Ala Ala Glu
   165 170 175
   Asn Val Ile Asp Val Lys Leu Val Asp Ala Asn Gly Lys Leu His Asp
   180 185 190
   Lys Lys Ser Met Gly Asp Asp His Phe Trp Ala Val Arg Gly Gly Gly
   195 200 205
   Gly Glu Ser Phe Gly Ile Val Val Ala Trp Gln Val Lys Leu Leu Pro
   210 215 220
   Val Pro Pro Thr Val Thr Ile Phe Lys Ile Ser Lys Thr Val Ser Glu
   225 230 235 240
   Gly Ala Val Asp Ile Ile Asn Lys Trp Gln Val Val Ala Pro Gln Leu
   245 250 255
   Pro Ala Asp Leu Met Ile Arg Ile Ile Ala Gln Gly Pro Lys Ala Thr
   260 265 270
   Phe Glu Ala Met Tyr Leu Gly Thr Cys Lys Thr Leu Thr Pro Leu Met
   275 280 285
   Ser Ser Lys Phe Pro Glu Leu Gly Met Asn Pro Ser His Cys Asn Glu
   290 295 300
   Met Ser Trp Ile Gln Ser Ile Pro Phe Val His Leu Gly His Arg Asp
   305 310 315 320
   Ala Leu Glu Asp Asp Leu Leu Asn Arg Asn Asn Ser Phe Lys Pro Phe
   325 330 335
   Ala Glu Tyr Lys Ser Asp Tyr Val Tyr Gln Pro Phe Pro Lys Thr Val
   340 345 350
   Trp Glu Gln Ile Leu Asn Thr Trp Leu Val Lys Pro Gly Ala Gly Ile
   355 360 365
   Met Ile Phe Asp Pro Tyr Gly Ala Thr Ile Ser Ala Thr Pro Glu Ser
   370 375 380
   Ala Thr Pro Phe Pro His Arg Lys Gly Val Leu Phe Asn Ile Gln Tyr
   385 390 395 400
   Val Asn Tyr Trp Phe Ala Pro Gly Ala Ala Ala Ala Pro Leu Ser Trp
   405 410 415
   Ser Lys Asp Ile Tyr Asn Tyr Met Glu Pro Tyr Val Ser Lys Asn Pro
   420 425 430
   Arg Gln Ala Tyr Ala Asn Tyr Arg Asp Ile Asp Leu Gly Arg Asn Glu
   435 440 445
   Val Val Asn Asp Val Ser Thr Tyr Ala Ser Gly Lys Val Trp Gly Gln
   450 455 460
   Lys Tyr Phe Lys Gly Asn Phe Glu Arg Leu Ala Ile Thr Lys Gly Lys
   465 470 475 480
   Val Asp Pro Thr Asp Tyr Phe Arg Asn Glu Gln Ser Ile Pro Pro Leu
   485 490 495
   Ile Lys Lys Tyr
   500
<210> 7
   <211> 10
   <212> PRT
   <213> Phleum pratense
<220>
   <221> MISC_FEATURE
   <222> (6)..(6)
   <223> undetermined amino acid
<400> 7
   Ile Val Ala Leu Pro Xaa Gly Met Leu Lys
   1 5 10
<210> 8
   <211> 14
   <212> PRT
   <213> Lolium perenne
<400> 8
   Phe Leu Glu Pro Val Leu Gly Leu Ile Phe Pro Ala Gly Val
   1 5 10
<210> 9
   <211> 9
   <212> PRT
   <213> Lolium perenne
<400> 9
   Gly Leu Ile Glu Phe Pro Ala Gly Val
   1 5
<210> 10
   <211> 12
   <212> PRT
   <213> Dactylus glomerata
<400> 10
   Asp Ile Tyr Asn Tyr Met Glu Pro Tyr Val Ser Lys
   1 5 10
<210> 11
   <211> 11
   <212> PRT
   <213> Dactylus glomerata
<400> 11
   Val Asp Pro Thr Asp Tyr Phe Gly Asn Glu Gln
   1 5 10
<210> 12
   <211> 17
   <212> PRT
   <213> Dactylus glomerata
<400> 12
   Ala Arg Thr Ala Trp Val Asp Ser Gly Ala Gln Leu Gly Glu Leu Ser
   1 5 10 15
   Tyr
<210> 13
   <211> 15
   <212> PRT
   <213> Dactylus glomerata
<400> 13
   Gly Val Leu Phe Asn Ile Gln Tyr Val Asn Tyr Trp Phe Ala Pro
   1 5 10 15
<210> 14
   <211> 11
   <212> PRT
   <213> Cynodon dactylon
<400> 14
   Lys Thr Val Lys Pro Leu Tyr Ile Ile Thr Pro
   1 5 10
<210> 15
   <211> 22
   <212> PRT
   <213> Cynodon dactylon
<400> 15
   Lys Gln Val Glu Arg Asp Phe Leu Thr Ser Leu Thr Lys Asp Ile Pro
   1 5 10 15
   Gln Leu Tyr Leu Lys Ser
   20
<210> 16
   <211> 16
   <212> PRT
   <213> Cynodon dactylon
<400> 16
   Thr Val Lys Pro Leu Tyr Ile Ile Thr Pro Ile Thr Ala Ala Met Ile
   1 5 10 15
<210> 17
   <211> 24
   <212> PRT
   <213> Cynodon dactylon
<400> 17
   Leu Arg Lys Tyr Gly Thr Ala Ala Asp Asn Val Ile Asp Ala Lys Val
   1               5                   10                  15
   Val Asp Ala Gln Gly Arg Leu Leu
               20
<210> 18
   <211> 14
   <212> PRT
   <213> Cynodon dactylon
<400> 18
   Lys Trp Gln Thr Val Ala Pro Ala Leu Pro Asp Pro Asn Met
   1               5                   10
<210> 19
   <211> 15
   <212> PRT
   <213> Cynodon dactylon
<400> 19
   Val Thr Trp Ile Glu Ser Val Pro Tyr Ile Pro Met Gly Asp Lys
   1               5                   10                  15
<210> 20
   <211> 19
   <212> PRT
   <213> Cynodon dactylon
<220>
   <221> MISC_FEATURE
   <222> (8)..(8)
   <223> undetermined amino acid
<400> 20
   Gly Thr Val Arg Gln Leu Leu Xaa Arg Thr Ser Asn Ile Lys Ala Phe
   1               5                   10                  15
   Gly Lys Tyr
<210> 21
   <211> 23
   <212> PRT
   <213> cynodon dactylon
<400> 21
   Thr Ser Asn Ile Lys Ala Phe Gly Lys Tyr Lys Ser Asp Tyr Val Leu
   1 5 10 15
   Glu Pro Ile Pro Lys Lys Ser
   20
<210> 22
   <211> 13
   <212> PRT
   <213> cynodon dactylon
<400> 22
   Tyr Arg Asp Leu Asp Leu Gly Val Asn Gln Val Val Gly
   1 5 10
<210> 23
   <211> 15
   <212> PRT
   <213> Cynodon dactylon
<400> 23
   Ser Ala Thr Pro Pro Thr His Arg Ser Gly Val Leu Phe Asn Ile
   1 5 10 15
<210> 24
   <211> 36
   <212> PRT
   <213> Cynodon dactylon
<400> 24
   Ala Ala Ala Ala Leu Pro Thr Gln Val Thr Arg Asp Ile Tyr Ala Phe
   1 5 10 15
   Met Thr Pro Tyr Val Ser Lys Asn Pro Arg Gln Ala Tyr Val Asn Tyr
   20 25 30
   Arg Asp Leu Asp
   35
<210> 25
   <211> 149
   <212> DNA
   <213> Phleum pratense
<400> 25
   caccggaagg gggtgctgtt caacatccag tacgtcaact actggttcgc cccgggagcc 60
   ggcgcggcgc cattgtcgtg gagcaaggag atctacaact acatggagcc gtacgtgagc 120
   aaggaccccg tccaggccta cgccaacta 149
<210> 26
   <211> 299
   <212> DNA
   <213> Phleum pratense
<400> 26
   actactggtt cgccccggga gccggcgcgg cgccattgtc gtggagcaag gagatctaca      60
   actacatgga gccatacgtg agcaagaacc ccaggcaggc ctacgccaac tacagggaca     120
   tcgacctcgg gaggaacgag gtggtgaacg acgtctccac cttcagcagc ggtttggtgt     180
   ggggccagaa atacttcaag ggcaacttcc agaggctcgc catcaccaag ggcaaggtgg     240
   atcccaccga ctacttcagg aacgagcaga gcatcccgcc gctcatcaaa aagtactga      299
<210> 27
   <211> 33
   <212> PRT
   <213> Phleum pratense
<220>
   <221> MISC_FEATURE
   <222> (14)..(14)
   <223> undetermined amino acid
<400> 27
   Tyr Phe Pro Pro Pro Ala Ala Lys Glu Asp Phe Leu Gly Xaa Leu Val
   1               5                   10                  15
   Lys Glu Ile Pro Pro Arg Leu Leu Tyr Ala Lys Ser Ser Pro Ala Tyr
               20                  25                  30
   Pro
<210> 28
   <211> 18
   <212> PRT
   <213> Phleum pratense
<220>
   <221> MISC_FEATURE
   <222> (6)..(6)
   <223> undetermined amino acid
<400> 28
   Ser Ala Thr Pro Phe Xaa His Arg Lys Gly Val Leu Phe Asn Ile Gln
   1               5                   10                  15
   Tyr Val
<210> 29
   <211> 10
   <212> PRT
   <213> Phleum pratense
<220>
   <221> MISC_FEATURE
   <222> (3)..(8)
   <223> undetermined amino acid
<400> 29
   Gly Leu Xaa Tyr Arg Xaa Leu Xaa Pro Glu
   1               5                   10
<210> 30
   <211> 12
   <212> PRT
   <213> Phleum pratense
<220>
   <221> MISC_FEATURE
   <222> (2)..(9)
   <223> undetermined amino acid
<400> 30
   Lys Xaa Met Gly Asp Asp His Phe Xaa Ala Val Arg
   1               5                   10
<210> 31
   <211> 9
   <212> PRT
   <213> Phleum pratense
<400> 31
   Ala Pro Glu Gly Ala Val Asp Ile Ile
   1               5
<210> 32
   <211> 16
   <212> PRT
   <213> Phleum pratense
<400> 32
   Met Glu Pro Tyr Val Ser Ile Asn Pro Val Gln Ala Tyr Ala Asn Tyr
   1               5                   10                  15
<210> 33
   <211> 15
   <212> PRT
   <213> Phleum pratense
<220>
   <221> MISC_FEATURE
   <222> (14)..(14)
   <223> undetermined amino acid
<400> 33
   Tyr Phe Pro Pro Pro Ala Ala Lys Glu Asp Phe Leu Gly Xaa Leu
   1               5                   10                  15
<210> 34
   <211> 10
   <212> PRT
   <213> Phleum pratense
<400> 34
   Leu Tyr Ala Lys Ser Ser Pro Ala Tyr Pro
   1               5                   10
<210> 35
   <211> 33
   <212> PRT
   <213> Phleum pratense
<220>
   <221> MISC_FEATURE
   <222> (14)..(14)
   <223> undetermined amino acid
<400> 35
   Tyr Phe Pro Pro Pro Ala Ala Lys Glu Asp Phe Leu Gly Xaa Leu Val
   1               5                   10                  15
   Lys Glu Ile Pro Pro Arg Leu Leu Tyr Ala Lys Ser Ser Pro Ala Tyr
               20                  25                  30
   Pro
<210> 36
   <211> 29
   <212> PRT
   <213> Phleum pratense
<220>
   <221> MISC_FEATURE
   <222> (14)..(14)
   <223> undetermined amino acid
<400> 36
   Tyr Phe Pro Pro Pro Ala Ala Lys Glu Asp Phe Leu Gly Xaa Leu Val
   1               5                   10                  15
   Lys Glu Pro Pro Arg Leu Leu Tyr Ala Lys Ser Ser Pro
               20                  25
<210> 37
   <211> 15
   <212> PRT
   <213> Phleum pratense
<220>
   <221> MISC_FEATURE
   <222> (4)..(14)
   <223> undetermined amino acid
<400> 37
   Tyr Phe Pro Xaa Xaa Ala Ala Lys Glu Asp Phe Leu Gly Xaa Leu
   1               5                   10                  15
<210> 38
   <211> 15
   <212> PRT
   <213> Phleum pratense
<220>
   <221> MISC_FEATURE
   <222> (4)..(14)
   <223> undetermined amino acid
<400> 38
   Tyr Phe Pro Xaa Xaa Ala Lys Lys Glu Asp Phe Leu Gly Xaa Leu
   1               5                   10                  15
<210> 39
   <211> 15
   <212> PRT
   <213> Phleum pratense
<220>
   <221> MISC_FEATURE
   <222> (4)..(14)
   <223> undetermined amino acid
<400> 39
   Tyr Phe Pro Xaa Xaa Ala Ala Lys Asp Asp Phe Leu Gly Xaa Leu
   1               5                   10                  15
<210> 40
   <211> 11
   <212> PRT
   <213> Phleum pratense
<220>
   <221> MISC_FEATURE
   <222> (4)..(5)
   <223> undetermined amino acid
<400> 40
   Tyr Phe Pro Xaa Xaa Leu Ala Asn Glu Asp Phe
   1               5                   10
<210> 41
   <211> 18
   <212> PRT
   <213> Phleum pratense
<220>
   <221> MISC_FEATURE
   <222> (6)..(6)
   <223> undetermined amino acid
<400> 41
   Ser Ala Thr Pro Phe Xaa His Arg Lys Gly Val Leu Phe Asn Ile Gln
   1               5                   10                  15
   Tyr Val
<210> 42
   <211> 10
   <212> PRT
   <213> Phleum pratense
<220>
   <221> MISC_FEATURE
   <222> (3)..(8)
   <223> undetermined amino acid
<400> 42
   Gly Leu Xaa Tyr Arg Xaa Leu Xaa Pro Glu
   1               5                   10
<210> 43
   <211> 12
   <212> PRT
   <213> Phleum pratense
<220>
   <221> MISC_FEATURE
   <222> (2)..(9)
   <223> undetermined amino acid
<400> 43
   Lys Xaa Met Gly Asp Asp His Phe Xaa Ala Val Arg
   1 5 10
<210> 44
   <211> 9
   <212> PRT
   <213> Phleum pratense
<400> 44
   Ala Pro Glu Gly Ala Val Asp Ile Ile
   1 5
<210> 45
   <211> 16
   <212> PRT
   <213> Phleum pratense
<400> 45
   Met Glu Pro Tyr Val Ser Ile Asn Pro Val Gln Ala Tyr Ala Asn Tyr
   1 5 10 15
<210> 46
   <211> 29
   <212> DNA
   <213> Phleum pratense
<220>
   <221> misc_feature
   <222> (1)..(29)
   <223> 'n' means inosin
<400> 46
   ytntaygcna arwsnwsncc ngcntaycc 29
<210> 47
   <211> 28
   <212> DNA
   <213> Phleum pratense
<220>
   <221> misc_feature
   <222> (1)..(28)
   <223> 'n' means inosin
<400> 47
   caymgnaarg gngtnytntt yaayatmc 28
<210> 48
   <211> 26
   <212> DNA
   <213> Phleum pratense
<220>
   <221> misc_feature
   <222> (1)..(26)
   <223> 'n' means inosin
<400> 48
   tarttngcrt angcytgnac nggrtt 26
<210> 49
   <211> 23
   <212> DNA
   <213> Phleum pratense
<400> 49
   actactggtt cgccccggga gcc 23
<210> 50
   <211> 28
   <212> DNA
   <213> Phleum pratense
<400> 50
   tgaagtattt ctggccccac accaaacc 28
<210> 51
   <211> 24
   <212> DNA
   <213> Phleum pratense
<400> 51
   cccttggtga tggcgagcct ctgg 24
<210> 52
   <211> 23
   <212> DNA
   <213> Phleum pratense
<400> 52
   ctcagtcctg gggcagacca tcc 23

## Beschreibung

### Hintergrund der Erfindung

Die vorliegende Erfindung betrifft die Bereitstellung der Gensequenz des Graspollenhauptallergens Phl p 4. Die Erfindung schließt auch Fragmente, Neukombinationen von Teilsequenzen und Punktmutanten mit hypoallergener Wirkung ein. Die rekombinanten DNA-Moleküle und die abgeleiteten Polypeptide, Fragmente, Neukombinationen von Teilsequenzen und Varianten können zur Therapie von pollenallergischen Krankheiten genutzt werden. Die rekombinant hergestellten Proteine können zur *in vitro-* und *in vivo*-Diagnostik von Pollenallergien eingesetzt werden.

Allergien vom Typ 1 haben weltweite Bedeutung. Bis zu 20 % der Bevölkerung in industrialisierten Ländern leiden unter Beschwerden wie allergischer Rhinitis, Konjunktivitis oder Bronchialasthma. Diese Allergien werden durch in der Luft befindliche Allergene (Aeroallergene), die von Quellen unterschiedlicher Herkunft wie Pflanzenpollen, Milben, Katzen oder Hunden freigesetzt werden, hervorgerufen. Bis zu 40 % dieser Typ 1-Allergiker wiederum zeigen spezifische IgE-Reaktivität mit Gräserpollenallergenen (Freidhoff et al., 1986, J. Allergy Clin. Immunol. 78, 1190-2001).

Bei den Typ 1-Allergie auslösenden Substanzen handelt es sich um Proteine, Glykoproteine oder Polypeptide. Diese Allergene reagieren nach Aufnahme über die Schleimhäute mit den bei sensibilisierten Personen an der Oberfläche von Mastzellen gebundenen IgE-Molekülen. Werden zwei IgE-Moleküle durch ein Allergen miteinander vernetzt, führt dies zur Ausschüttung von Mediatoren (z. B. Histamin, Prostaglandine) und Zytokinen durch die Effektorzelle und damit zu den entsprechenden klinischen Symptomen.

In Abhängigkeit von der relativen Häufigkeit mit der die einzelnen Allergenmoleküle mit den IgE-Antikörpern von Allergikern reagieren, wird zwischen Major- und Minorallergenen unterschieden.

Im Fall vom Wiesenlieschengras (*Phleum pratense*) sind bislang Phl p 1 (Petersen et al., 1993, J. Allergy Clin. Immunol. 92: 789-796), Phl p 5 (Matthiesen und Löwenstein, 1991, Clin. Exp. Allergy 21: 297-307; Petersen et al., 1992, Int. Arch. Allergy Immunol. 98: 105-109), Phl p 6 (Petersen et al., 1995, Int. Arch. Allergy Immunol. 108, 49-54). Phl p 2/3 (Dolecek et al., 1993, FEBS 335 (3), 299-304), Phl p 4 (Haavik et al., 1985, Int. Arch. Allergy Appl. Immunol. 78: 260-268; Valenta et al., 1992, Int. Arch. Allergy Immunol. 97: 287-294, Fischer et al., 1996, J. Allergy Clin. Immunol. 98: 189-198) und Phl p 13 (Suck et al., 2000, Clin. Exp. Allergy 30: 324-332; Suck et al., 2000, Clin. Exp. Allergy 30: 1395-1402) als Hauptallergene identifiziert worden.

Das Phl p 4 ist als basisches Glykoprotein mit einer Molekularmasse zwischen 50 und 60 kDa angegeben worden (Haavik et al., 1985, Int. Arch. Allergy Appl. Immunol. 78: 260-268). Das Phl p 4-Molekül ist Trypsinresistent (Fischer et al., 1996, J. Allergy Clin. Immunol. 98: 189-198) und 70-88 % der Graspollenallergiker haben IgE-Antikörper gegen dieses Molekül (Valenta et al., 1993, Int. Arch. Allergy Immunol. 97: 287-294; Rossi et al., 2001, Allergy 56:1180-1185; Mari, 2003, Clin. Exp. Allergy 33:43-51). Homologe Moleküle sind aus verwandten Grasspezies beschrieben worden (Su et al., 1991, Clin. Exp. Allergy 21: 449-455; Jaggi et al., 1989, Int. Arch. Allergy Appl. Immunol. 89: 342-348; Jaggi et al., 1989, J. Allergy Clin. Immunol. 83: 845-852; Leduc-Brodard et al., 1996, J. Allergy Clin. Immunol. 98: 1065-1072; 14 - 17). Diese homologen Moleküle der *Poaceae* bilden die Allergengruppe 4, deren Moleküle eine hohe immunologische Kreuzreaktivität untereinander sowohl mit monoklonalen Mausantikörpern als auch mit humanen IgE-Antikörpern aufweisen (Fahlbusch et al., 1993 Clin. Exp. Allergy 23:51-60; Leduc-Brodard et al., 1996, J. Allergy Clin. Immunol. 98:1065-1072; Su et al., 1996, J. Allergy Clin. Immunol. 97:210; Fahlbusch et al., 1998, Clin. Exp. Allergy 28:799-807; Gavrovic-Jankulovic et al., 2000, Invest. Allergol. Clin. Immunol. 10 (6): 361-367; Stumvoll et al. 2002, Biol. Chem. 383: 1383-1396; Grote et al., 2002, Biol. Chem. 383: 1441-1445; Andersson und Lidholm, 2003, Int. Arch. Allergy Immunol. 130: 87-107; Mari, 2003, Clin. Exp. Allergy, 33 (1): 43-51).
Im Gegensatz zu den oben genannten Hauptallergenen von *Phleum pratense* (Phl p 1, Phl p 2/3, Phl 5a und 5b, Phl p 6 und Phl p 13) ist die Primärstruktur des Phl p 4 noch nicht aufgeklärt. Ebenso gibt es keine vollständige Sequenz von Molekülen der Gruppe 4 aus anderen Grasspezies.

Die Bestimmung der N-terminalen Aminosäuresequenz war bisher nicht erfolgreich. Die Ursachen hierfür sind jedoch nicht bekannt. Fischer et al. (J. Allergy Clin. Immunol., 1996; 98: 189-198) vermuten eine N-terminale Blockierung, konnten aber ein internes Peptid nach Abbau mit Lysyl-Endopeptidase reinigen und dessen Sequenz bestimmen: IVALPXGMLK (SEQ ID NO 7).
Dieses Peptid weist Homologien zu Peptidsequenzen in den Ragweed-Allergenen Amb a1 und Amb a2 sowie Ähnlichkeiten zu Sequenzen in Proteinen von Mais (Zm58.2), Tomate (lat 59, lat 56) und Tabak (G10) auf (Fischer et al., 1996, J. Allergy Clin. Immunol. 98: 189-198). Für *Lolium perenne* wurden für das basische Gruppe-4 Allergen Peptidfragmente mit der folgenden Sequenz beschrieben: FLEPVLGLIFPAGV (SEQ ID NO 8) und GLIEFPAGV (SEQ ID NO 9) (Jaggi et al., 1989, Int. Arch. Allergy Appl. Immunol. 89: 342-348).

Von dem Gruppe-4 Allergen aus *Dactylus glomerata* sind ebenfalls Peptide durch enzymatischen Abbau gewonnen und sequenziert worden:
DIYNYMEPYVSK (P15, SEQ ID NO 10),
VDPTDYFGNEQ (P17, SEQ ID NO 11),
ARTAWVDSGAQLGELSY (P20, SEQ ID NO 12) und GVLFNIQYVNYWFAP (P22, SEQ ID NO 13) (Leduc-Brodard et al., 1996, J. Allergy Clin. Immunol. 98: 1065-1072).
Auch vom Gruppe-4 Allergen des subtropischen Bermuda-Grases (*Cynodon dactylon*) sind durch Proteolyse Peptide erhalten und sequenziert worden:
KTVKPLYIITP (S, SEQ ID NO 14),
KQVERDFLTSLTKDIPQLYLKS (V49L, SEQ ID NO 15),
TVKPLYIITPITAAMI (T33S, SEQ ID NO 16),
LRKYGTAADNVIDAKVVDAQGRLL (T35L, SEQ ID NO 17),
KWQTVAPALPDPNM (P2, SEQ ID NO 18),
VTWIESVPYIPMGDK (V26L, SEQ ID NO 19),
GTVRDLLXRTSNIKAFGKY (L25L, SEQ ID NO 20),
TSNIKAFGKYKSDYVLEPIPKKS (T22L, SEQ ID NO 21),
YRDLDLGVNQVVG (P3, SEQ ID NO 22),
SATPPTHRSGVLFNI (V20L, SEQ ID NO 23),
und AAAALPTQVTRDIYAFMTPYVSKNPRQAYVNYRDLD (V14L, SEQ ID NO 24) (Liaw et al., 2001, Biochem. Biophys. Research Communication 280: 738-743).

Diese beschriebenen Peptidsequenzen für Phl p 4 und Gruppe-4 Allergene haben jedoch bisher nicht zur Aufklärung der vollständigen Primärstruktur der Gruppe-4 Allergene geführt.

Die der vorliegenden Erfindung zugrunde liegende Aufgabe bestand daher in der Bereitstellung der vollständigen DNA-Sequenz des Phl p 4 sowie einer entsprechenden rekombinanten DNA, auf deren Grundlage das Phl p 4-Allergen als Protein exprimiert und einer pharmakologisch bedeutsamen Verwertung als solches oder in veränderter Form zugänglich gemacht werden kann.

### Verzeichnis der Abbildungen

**Abbildung 1:** Interne DNA-Sequenz (SEQ ID NO 25) des Phl p 4-Gens Mit den degenerierten Primern Nr. 30 (sense) und Nr. 37 (anti-sense), beide kursiv dargestellt, wurden mit genomischer DNA erhaltene Amplifikate kloniert und sequenziert. Die dargestellte Sequenz repräsentiert den Konsensus aus 6 Klonen. Der aus dieser Sequenz kreierte spezifische sense-Primer Nr. 82 ist unterstrichen dargestellt.

**Abbildung 2:** 3'-Ende der Nukleinsäuresequenz (SEQ ID NO 26) des Phl p 4-Gens
In einer 3'-RACE PCR mit *Phleum pratense* cDNA wurden Amplifikate mit dem spezifischen sense-Primer Nr. 82 (kursiv dargestellt) sowie einem Ankerprimer gewonnen und sequenziert. Die dargestellte Sequenz repräsentiert den Konsensus aus 3 Sequenzierungen und umfasst das 3'-Ende des Phl p 4-Gens bis zum Stopp-Codon (doppelt unterstrichen). Die Sequenzbereiche, die zur Konstruktion der anti-sense-Primer Nr. 85 und Nr. 86 herangezogen wurden, sind unterstrichen dargestellt.

**Abbildung 3:** Lokalisierung der Phl p 4- Peptide in der deduzierten Aminosäuresequenz des Phl p 4-Allergens (SEQ ID NO 2)
Die aus der Aminosäuresequenzierung des gereinigten und fragmentierten Phl p 4 Allergens erhaltenen Peptide P1 - P6 (SEQ ID NO's 27-32) lassen sich eindeutig der aus der Nukleinsäuresequenz abgeleiteten Aminosäuresequenz des Phl p 4-Gens zuordnen.

**Abbildung 4:** Nachweis der Identität des rekombinanten Phl p 4 (rPhl p 4) mittels der für nPhl p 4 spezifischen monoklonalen Antikörper 5H1 (Blot A) und 3C4 (Blot B) im Western Blot.
Bahn 1: *E. coli* Gesamtzellextrakt enthaltend rPhl p 4 Fragment 1-200
Bahn 2: E. *coli* Gesamtzellextrakt enthaltend rPhl p 4 Fragment 185-500
Bahn 3: *E. coli* Gesamtzellextrakt enthaltend rPhl p 4
Bahn 4: gereinigtes nPhl p 4 aus *Phleum pratense* (◄········): Abbruch- oder Degradationsfragmente von C-terminalem rPhl p 4-Fragment bzw. rPhl p 4 Gesamtmolekül

**Abbildung 5:** Nachweis der Reaktivität des rekombinanten Phl p 4 (rPhl p 4) mit IgE aus Seren von Graspollenallergikern im Western Blot. Extrakte von transformierten E. *coli*-Zellen, die entweder das komplette Phl p 4 Gen exprimieren oder das N-terminale Fragment 1-200 bzw. das C-terminale Fragment 185-500 wurden in der SDS-PAGE getrennt und auf Nitrozellulosemembranen transferiert. Der Blot wurde mit Seren der graspollenallergischen Spender A, B oder C inkubiert und nachfolgend gebundenes IgE über einen anti-human-IgE Antikörper, konjugiert mit alkalischer Phosphatase, kolorimetrisch detektiert.
Bahn 1: *E*. *coli* Gesamtzellextrakt enthaltend rPhl p 4 Fragment 1-200
Bahn 2: *E. coli* Gesamtzellextrakt enthaltend rPhl p 4 Fragment 185-500
Bahn 3: *E*. *coli* Gesamtzellextrakt enthaltend rPhl p 4
Bahn 4: gereinigtes nPhl p 4 aus *Phleum pratense*

Die vor- und nachstehend verwendeten Bezeichnungen für Nukleotid- bzw. Aminosäuresequenzen "SEQ ID NO" beziehen sich auf das der Beschreibung beigefügte Sequenzprotokoll.

### Beschreibung der Erfindung

Mit der vorliegenden Erfindung wird die Gensequenz des Graspollenhauptallergens Phl p 4 nun erstmals bereit gestellt, wobei sich aus den aufgefundenen Einzelnukleotidaustauschen (Single Nucleotide Polymorphisms, SNP's) drei dominierende Sequenzen (SEQ ID NO 1, 3 und 5) ergeben.

Gegenstand der vorliegenden Erfindung ist daher ein DNA-Molekül mit der Nukleotidsequenz SEQ ID NO 1 mit einem Einzelnukleotidaustausch ausgewählt aus der Gruppe bestehend aus T85A, C130A, G159A, A160C, G169A, G222C, G226A, G227C, T228C, C237T, C285T, C286T, G298A, A299C, C303T, C309G, T318C, G320A, C333G, G348C, C369G, C409T, C411T, T420C, A421C, A423C, T425C, G522C, C525G, C618T, G657A, G662A, G684C, C690A, G691A, G693A, C703A, A710C, G711A, G743A, G750A, C768T, G798C, G801A, C804G, G834C, A865G, G879C, G918A, A994T, C1047A, A1051G, G1052A, G1053A, C1090T, G1098C, C1185G, A1278C, G1370C, C1414G, C1425A, C1428T, G1443C, C1449T und G1464A.

Im Zusammenhang mit der vorliegenden Erfindung sind außer den Gruppe-4 Allergenen der anderen Grasspezies die Gruppe-13 Allergene von Interesse, da sie ein den Gruppe-4 Allergenen sehr ähnliches Molekulargewicht in der SDS-PAGE zeigen und schwer durch biochemische Techniken abzutrennen sind (Suck et al., 2000, Clin. Exp. Allergy 30: 324-332, Suck et al., 2000, Clin. Exp. Allergy 30: 1395-1402). Mit Hilfe der jetzt erstmalig vorliegenden, erfindungsgemäßen Protein- und DNA-Sequenz kann jedoch eindeutig gezeigt werden, dass die Gruppen 4 und 13 deutlich unterschiedliche Aminosäuresequenzen aufweisen.

Mit der Kenntnis der DNA-Sequenz der natürlich vorkommenden Allergene ist es nun möglich, diese Allergene als rekombinante Proteine herzustellen, die in der Diagnostik und Therapie von allergischen Erkrankungen Verwendung finden können (Scheiner and Kraft, 1995, Allergy 50: 384-391).

Ein klassischer Ansatz zur wirksamen therapeutischen Behandlung von Allergien stellt die Spezifische Immuntherapie oder Hyposensibilisierung dar (Fiebig, 1995, Allergo J. 4 (6): 336-339, Bousquet et al., 1998, J. Allergy Clin. Immunol. 102(4): 558-562). Dabei werden dem Patienten natürliche Allergenextrakte in steigenden Dosen subkutan injiziert. Allerdings besteht bei dieser Methode die Gefahr von allergischen Reaktionen oder sogar eines anaphylaktischen Schocks. Um diese Risiken zu minimieren, werden innovative Präparate in Form von Allergoiden eingesetzt. Dabei handelt es sich um chemisch modifizierte Allergenextrakte, die deutlich reduzierte IgE-Reaktivität, jedoch identische T-Zell-Reaktivität im Vergleich zum nicht behandelten Extrakt aufweisen (Fiebig, 1995, Allergo J. 4 (7): 377-382).
Eine noch weitergehende Therapieoptimierung wäre mit rekombinant hergestellten Allergenen möglich. Definierte, ggfs. auf die individuellen Sensibilisierungsmuster der Patienten abgestimmte Cocktails von hochreinen, rekombinant hergestellten Allergenen könnten Extrakte aus natürlichen Allergenquellen ablösen, da diese außer den verschiedenen Allergenen eine größere Zahl von immunogenen, aber nicht allergenen Begleitproteinen enthalten.
Realistische Perspektiven, die zu einer sicheren Hyposensibilisierung mit Expressionsprodukten führen können, bieten gezielt mutierte rekombinante Allergene, bei denen IgE-Epitope spezifisch deletiert werden, ohne die für die Therapie essentiellen T-Zell Epitope zu beeinträchtigen (Schramm et al., 1999, J. Immunol. 162: 2406-2414).

Eine weitere Möglichkeit zur therapeutischen Beeinflussung des gestörten TH-Zell-Gleichgewichtes bei Allergikern ist die immuntherapeutische DNA-Vakzinierung. Dabei handelt es sich um eine Behandlung mit expressionsfähiger DNA, die für die relevanten Allergene kodiert. Erste experimentelle Belege für die allergenspezifische Beeinflussung der Immunantwort konnte an Nagern durch Injektion von Allergen-kodierender DNA erbracht werden (Hsu et al., 1996, Nature Medicine 2 (5): 540-544).

Die entsprechenden rekombinant hergestellten Proteine können zur Therapie sowie zur *in vitro-* und *in vivo*-Diagnostik von Pollenallergien eingesetzt werden.
Zur Herstellung des rekombinanten Allergens wird die klonierte Nukleinsäure in einen Expressionsvektor ligiert und dieses Konstrukt in einem geeigneten Wirtsorganismus exprimiert. Nach biochemischer Reinigung steht dieses rekombinante Allergen zur Detektion von IgE-Antikörpern in etablierten Verfahren zur Verfügung.

Gegenstand der vorliegenden Erfindung ist daher weiterhin ein rekombinanter Expressionsvektor, enthaltend ein vor- oder nachstehend beschriebenes DNA-Molekül, funktionell verbunden mit einer Expressionskontrollsequenz und ein Wirtsorganismus, transformiert mit besagtem DNA-Molekül oder besagtem Expressionsvektor.

Wie bereits ausgeführt kann die Erfindung als eine essentielle Komponente in einem rekombinanten allergen- oder nukleinsäurehaltigen Präparat zur spezifischen Immuntherapie angewendet werden. Hierbei bieten sich mehrere Möglichkeiten. Zum einen kann das in der Primärstruktur unveränderte Protein Bestandteil des Präparates sein. Zum anderen kann durch gezielte Deletion von IgE-Epitopen des Gesamtmoleküls oder der Herstellung von einzelnen Fragmenten, die für T-Zell Epitope kodieren, erfindungsgemäß eine hypoallergene (allergoide) Form zur Therapie verwendet werden, um unerwünschte Nebenwirkungen zu vermeiden. Schließlich wird durch die Nukleinsäure an sich, wenn sie mit einem eukaryontischen Expressionsvektor ligiert wird, ein Präparat geschaffen, das direkt appliziert den allergischen Immunzustand im therapeutischen Sinne verändert.

Die vorliegende Anmeldung offenbart somit rekombinante DNA-Moleküle entsprechend den SEQ ID NO 1, 3 oder 5, wobei die Nukleotidsequenz der Positionen 1-69 aus der Aminosäuresequenz des Phl p 4 N-Terminus abgeleitet wurde. Dabei wurden in *E. coli* häufig vorkommende Codons verwendet. Ab Position 70 entspricht die DNA-Sequenz derjenigen, die in genomischer und cDNA von *Phleum pratense* identifiziert wurde.
Die vorliegende Anmeldung offenbart daher weiterhin ein DNA-Molekül, umfassend eine Nukleotidsequenz gemäß SEQ ID NO 1, SEQ ID NO 3 bzw. SEQ ID NO 5, beginnend mit der Position 70, welches für ein Polypeptid mit den Eigenschaften des Majorallergens Phl p 4 aus *Phleum pratense* kodiert.

Gegenstand der Erfindung ist somit auch ein Polypeptid mit der Aminosäuresequenz SEQ ID NO 2 mit einem Aminosäureaustausch ausgewählt aus der Gruppe bestehend aus A6L, A7K, K8N, E9D, S29T, I54L, V57I, G76T, E100T, S107N, H137Y, T141P, V142A, R221K, V231I, P235T, K237T, R248K, S289G, T332S, R351E, P364S, Q472E und K498Q.

Die vorliegende Anmeldung offenbart auch Polypeptide gemäß SEQ ID NO 2, SEQ ID NO 4 bzw. SEQ ID NO 6, wobei die Aminosäurepositionen 1-33 durch N-terminale Aminosäuresequenzierung des isolierten natürlichen Phl p 4 Allergens bestimmt wurden. Die Positionen 24-500 wurden von der DNA-Sequenz gemäß SEQ ID NO 1, 3 bzw. 5 abgeleitet. Variable Aminosäuren an den Positionen 6, 7, 8 und 9 entstammen der N-terminalen Proteinsequenzierung unterschiedlicher Präparationen des natürlichen Phl p 4 (Tab. 1).

Diese erfindungsgemäßen Polypeptide bzw. Proteine, welche für Menschen als Allergene wirken, sind in den Pollenkörnern von *Phleum praten*se enthalten. Die Pollenkörner der anderen Poaceae-Spezies, wie z.B. *Lolium perenne, Dactylis glomerata, Poa pratensis, Cynodon dactylon, Holcus lanatus* u.a. enthalten homologe Allergenmoleküle (Gruppe-4 Allergene).
Die Homologie dieser Moleküle ist durch ihre immunologische Kreuzreaktivität sowohl mit murinen monoklonalen Antikörpern als auch mit humanen IgE-Antikörpern nachgewiesen worden.

Bei der Ermittlung der Protein- und DNA-Sequenz des Phl p 4 wurde wie folgt vorgegangen:
Die Reinigung und Isolierung des natürlichen Allergens Phl p 4 erfolgte nach beschriebenen Methoden (Fahlbusch et al. 1998, Clin. Exp. Allergy 28: 799-807, Suck et al. 2000, Clin. Exp. Allergy 30: 1395-1402). Die Feinreinigung und die Abtrennung von Spuren des Allergens der Gruppe 13 erfolgte nach der von Suck et al. (2000, Clin. Exp. Allergy 30: 1395-1402) beschriebenen Methode.
Von diesem aus *Phleum pratense* isolierten Phl p 4 wurde die N-terminale Aminosäuresequenz mittels Edman-Abbau bestimmt. Mit unterschiedlichen Chargen des Phl p 4 wurden die in Tabelle 1 aufgezeigten N-terminalen Sequenzen (P1a - f) bestimmt. Als Konsensussequenz für die ersten 15 Positionen wird folgende Sequenz angesehen: YFPP'P'AAKEDFLGXL (SEQ ID NO 33). Die Position 14 konnte nicht bestimmt werden, wahrscheinlich ist sie mit einem Cystein besetzt. Die unterschiedlichen Aminosäuren auf den Positionen 6, 7, 8 und 9 bei den unterschiedlichen Chargen weisen auf Variationen im Sinne von Isoformen hin. Die Positionen 4 und 5 sind mit Hydroxyprolin (P') besetzt, was durch eine spezifische Analytik bei den Analysen der Präparate p1-a und -b eindeutig festgestellt wurde.

Durch Behandlung des SDS-denaturierten Phl p 4 mit der Endopeptidase Glu-C (Promega, Heidelberg, Germany) wurden verschiedene Peptide erhalten. Von zwei Peptiden (P2 und P3) wurden die in Tabelle 1 gezeigten Aminosäuresequenzen bestimmt. Durch Spaltung mit der Endopeptidase Lys-C (Roche, Mannheim, Deutschland) wurden 2 Peptide (P4 und P5) gereinigt und sequenziert (Tab. 1). Durch CNBr-Spaltung wurde ein weiteres Peptid (P6) isoliert und die Aminosäuresequenz ermittelt (Tab.1).

Die Aminosäuresequenzen der N-terminalen Sequenz und der internen Peptide 2 und 6 wurden als Grundlage für die Konstruktion von degenerierten Primern benutzt. Mit dem Sense-Primer Nr. 30 und dem Anti-Sense-Primer Nr. 37 (Tab. 2) wurden mit genomischer DNA aus *Phleum pratense* Amplifikate hergestellt. Die von diesen Amplifikaten gewonnenen Klone wurden sequenziert (Abb. 1) und zur Konstruktion des spezifischen Sense-Primers Nr. 82 benutzt (Tab. 2). Mit einer cDNA, die aus der repräsentativen mRNA-Population aus *Phleum pratense*-Pollen hergestellt wurde, und dem erfindungsgemäßen spezifischen Sense-Primer Nr. 82 sowie dem Ankerprimer AUAP (Life Technologies, Karlsruhe, Deutschland) wurde eine PCR unter stringenten Bedingungen durchgeführt. Dieses Amplifikat von ca. 450 kb wurde sequenziert und somit die fehlende Sequenz bis zum 3'-Ende des Phl p 4-Genes identifiziert (Abb. 2). Basierend auf dieser erfindungsgemäß bestimmten C-terminalen Phl p 4-Sequenz wurden die spezifischen Anti-Sense-Primer Nr. 85 und Nr. 86 konstruiert (Tab. 2). Basierend auf der N-terminalen Aminosäuresequenz des Phl p 4-Peptids P1-a (Tab. 1) wurde der degenerierte Sense-Primer Nr. 29, abgeleitet von der DNA kodierend für die Aminosäurepositionen 24-33 (LYAKSSPAYP (SEQ ID NO 34)), konstruiert.

Mit den Primern Nr. 29 und Nr. 86 wurde mit genomischer *Phleum praten*se-DNA eine PCR durchgeführt. Dieses PCR-Produkt wurde als Grundlage für eine zweite PCR (nested PCR) mit den Primern Nr. 29 und Nr. 85 eingesetzt. Die Amplifikate wurden in den Vektor pGEM T-easy (Promega, Heidelberg, Deutschland) inseriert, kloniert und sequenziert. Diese Sequenz beginnt an der Position 24 vom N-Terminus gerechnet bzw. Position 70 der DNA-Sequenz gemäß SEQ ID NO 1, 3 oder 5 und reicht bis zum Primer Nr. 85, (Position 1402 in SEQ ID NO 1, 3 oder 5) der im schon bestimmten C-terminalen Abschnitt des Phl p 4-Genes lokalisiert ist. Mit diesen Daten kann die vollständige Aminosäuresequenz des Phl p 4-Moleküles aus den durch Proteinsequenzierung bestimmten ersten 33 Aminosäurepositionen und der deduzierten Aminosäuresequenz (477 Positionen), die aus den mit den Primern Nr. 29/Nr. 85 und Nr. 82/Anker-Primer hergestellten Klonen abgeleitet werden kann, konstruiert werden. Die beiden Klone überlappen in 197 Positionen ihrer Nukleotidsequenz. Das vom Klon Nr. 29/Nr. 85 kodierte Peptid überlappt auf 10 Aminosäurepositionen mit der durch direkte Aminosäuresequenzierung bestimmten N-terminalen Sequenz (Positionen 1-33) des Phl p 4, wobei die mit beiden Methoden bestimmten Aminosäuren übereinstimmen.

Die Aminosäuresequenz des Phl p 4 basierend auf den direkt bestimmten N-terminalen Aminosäuren und der deduzierten Aminosäuresequenz entspricht den unter den SEQ ID NO 2, 4 bzw. 6 im Sequenzprotokoll aufgeführten Sequenzen.

Mit dem spezifischen Sense-Primer Nr. 88 (Tab. 2) und dem spezifischen Anti-Sense-Primer Nr. 86 wurden sowohl mit genomischer als auch mit cDNA von *Phleum pratense* PCR-Produkte hergestellt und direkt sequenziert.
Hierdurch ist es möglich, PCR-Fehler auszuschliessen und genetische Variationen (single nucleotide polymorphisms) aufzudecken.
Die gefundenen Einzelnukleotidaustausche zur DNA-Sequenz SEQ ID NO 1 sind in Tabelle 3 aufgeführt. Einige dieser Einzelnukleotidaustausche führen zu veränderten Aminosäuren. Diese sind in Tabelle 4 dargestellt. Es wurden weiterhin DNA-Klone sequenziert, die zu abweichenden Aminosäuren in Bezug auf die dominierenden Sequenzen SEQ ID NO 2, 4 und 6 führen (Tab. 5).
Diese Aminosäurevariationen sind als Isoformen des Phl p 4-Moleküles anzusehen. Die Existenz solcher Isoformen ist aufgrund des heterogenen isoelektrischen Verhaltens des natürlichen Phl p 4 zu erwarten. Alle bisher bekannten Pollenallergene weisen solche Isoformen auf. Dass das DNA-Stück, welches mit den Primern Nr. 29 und 86 bestimmt wurde, tatsächlich für ein Protein kodiert, welches mit dem natürlichen Phl p 4-Allergen identisch ist, kann unter anderem auch dadurch bewiesen werden, dass für die identifizierten internen Peptide P3, P4 und P5 (Tab. 1) des natürlichen Phl p 4 homologe Peptidsequenzen in der deduzierten Aminosäuresequenz des erfindungsgemäßen rekombinanten Phl p 4-Moleküls gefunden werden (Abb. 3). Die beschriebene Aminosäuresequenz des Phl p 4 zeigt, dass es sich um ein basisches Molekül mit einem kalkulierten isoelektrischen Punkt von 8,99 (SEQ ID NO 2), 8,80 (SEQ ID NO 4) bzw. 9,17 (SEQ ID NO 6), bestehend aus 500 Aminosäuren, handelt. Die quantitative Aminosäurezusammensetzung ist in Tabelle 6 dargestellt. Das berechnete Molekulargewicht des rekombinanten Phl p 4 beträgt 55.762 (SEQ ID NO 2), 55.734 (SEQ ID NO 4) bzw. 55.624 (SEQ ID NO 6) Dalton. Diese berechnete Molekularmasse stimmt sehr gut mit der durch SDS-PAGE bestimmten Molekularmasse des natürlichen Phl p 4, für das 55 kDa bestimmt wurden, überein (Fahlbusch et al., 1998, Clin. Exp. Allergy 28: 799 -807 und Suck et al., 2000, Clin. Exp. Allergy 30: 1395-1402).

Auch für die Gruppe-4 Allergene verwandter Grasspezies wurden Molekularmassen zwischen 50 und 60 kDa beschrieben (Su et al., 1991, Clin. Exp. Allergy 21: 449-455; Jaggi et al., 1989, Int. Arch. Allergy Appl. Immunol. 89: 342-348; Jaggi et al., 1989, J. Allergy Clin. Immunol. 83: 845-852; Leduc-Brodard et al., 1996, J. Allergy Clin. Immunol. 98: 1065-1072; 14 - 17).

Zur Herstellung des rekombinanten Phl p 4-Proteins wurde die für Phl p 4-kodierende DNA-Sequenz gemäß SEQ ID NO 1, 3 und/oder 5 in Expressionsvektoren (z.B. pProEx, pλCro, pSE 380) eingebaut. Für die aus der Proteinsequenzierung bekannten N-terminalen Aminosäuren wurden *E*. *coli* optimierte Codons verwendet.

Nach der Transformation in *E*. *coli,* der Expression und der Reinigung des rekombinanten Phl p 4 durch verschiedene Trenntechniken wurde das erhaltene Protein einem Refoldingprozess unterworfen.
Dieses so gewonnene rPhl p 4-Protein ergibt eine einzelne Bande in der SDS-PAGE, die im gleichen Molekulargewichtsbereich wie das natürliche Phl p 4 wandert. Die immunologische Reaktivität des rPhl p 4 konnte durch die Reaktion mit den murinen monoklonalen Antikörpern 5H1 und 3C4, die mit natürlichem Phl p 4 induziert worden waren und mit den homologen Proteinen (Gruppe 4) der *Poaceae* kreuzreagieren (Fahlbusch et al., 1998, Clin. Exp. Allergy 28:799-807; Gavrović-Jankulović et al., 2000, Invest. Allergol. Clin. Immunol. 10 (6): 361-367), nachgewiesen werden (Abb. 4). Das rPhl p 4 reagiert mit IgE-Antikörpern von Allergikern, die nachgewiesene IgE-Reaktivität mit natürlichem Phl p 4 haben. Diese IgE-Reaktivität und damit die Wirkung als Allergen konnte sowohl im Dot-Test, Western-Blot als auch nach Adsorption des Allergens an Polystyren-Mikrotiterplatten nachgewiesen werden. Der Nachweis im Western-Blot ist in Abbildung 5 gezeigt. Bei Reaktion des rPhl p 4 mit Basophilen von Allergengruppe 4-reaktiven Graspollenallergikern werden diese zur verstärkten Expression des Aktivierungsmarkers CD 203c angeregt. Diese Basophilenaktivierung durch rPhl p 4 zeigt deutlich, dass dieses Molekül auch funktionell als Allergen wirkt.
Damit kann dieses rPhl p 4-Allergen zur hochspezifischen Diagnostik von Graspollenallergikern eingesetzt werden. Diese Diagnostik kann *in vitro* durch die Detektion von spezifischen Antikörpern (IgE, IgG1 - 4, IgA) und die Reaktion mit IgE-beladenen Effektorzellen (z. B. Basophile aus dem Blut) oder *in vivo* durch Hauttest-Reaktionen und Provokation am Reaktionsorgan erfolgen.

Die Reaktion des rPhl p 4 mit T-Lymphozyten von Graspollenallergikern konnte durch die allergenspezifische Stimulierung der T-Lymphozyten zur Proliferation und Zytokinsynthese sowohl mit T-Zellen in frisch präparierten Blutlymphozyten als auch an etablierten nPhl p 4-reaktiven T-Zell-Linien und -Klonen nachgewiesen werden.

Basierend auf der dargelegten DNA-Sequenz des rPhl p 4 wurden Teilsequenzen kodierend für Peptide von 50 bis 350 Aminosäuren in Expressionsvektoren kloniert. Diese Teilsequenzen decken sequentiell die vollständige Sequenz des rPhl p 4 ab, wobei Überlappungen von mindestens 12 Aminosäuren vorkommen. Die exprimierten Peptide entsprechen Phl p 4-Fragmenten. Diese Phl p 4-Fragmente reagieren einzeln oder als Gemisch mit den IgE-Antikörpern von Allergikern nicht oder nur in geringem Grade, so dass sie als hypoallergen eingestuft werden können. Im Gegensatz dazu ist das Gemisch dieser Fragmente in gleicher Weise wie das vollständige rekombinante oder natürliche Phl p 4 zur Stimulierung von T-Lymphozyten von Graspollenallergikern mit Phl p 4-Reaktivität fähig. Abbildung 4 zeigt als Beispiel die Charakterisierung zweier solcher Phl p 4-Fragmente, entsprechend den Aminosäuren 1-200 und 185-500 durch die Bindung an Phl p 4-spezifische monoklonale Mausantikörper. Das C-terminale Fragment 185-500 reagiert nur mit dem nonoklonalen Antikörper 5H1, während das N-terminale Fragment 1-200 eindeutig mit dem monoklonalen Antikörper 3C4 reagiert. Aus Abbildung 5 wird ersichtlich, daß das Fragment 185-500 weniger stark mit dem IgE aus den Seren der Allergiker B und C reagiert, also weniger allergen ist als das Fragment 1-200, welches zumindest gegenüber dem Patientenserum C eine herabgesetzte IgE-Reaktivität (Hypoallergenität) aufweist.

Durch ortsgerichte Mutagenese wurden die für die Cysteine kodierenden Tripletts so verändert, dass sie für andere Aminosäuren, bevorzugt Serin, kodieren. Es wurden sowohl Varianten hergestellt, bei denen einzelne Cysteine ausgetauscht wurden, als auch solche, bei denen verschiedene Kombinationen von 2 Cysteinresten bzw. alle 5 Cysteine verändert wurden. Die exprimierten Proteine dieser Cysteinpunktmutanten weisen eine stark reduzierte bzw. fehlende Reaktivität mit IgE-Antikörpern von Allergikern auf, reagieren jedoch mit den T-Lymphozythen dieser Patienten. Gegenstand der vorliegenden Erfindung ist daher weiterhin ein vor- oder nachstehend beschriebenes DNA-Molekül, bei dem durch ortsgerichtete Mutagenese einer, mehrere oder alle der Cystein-Reste des entsprechenden Polypeptids gegen eine andere Aminosäure ausgetauscht wurden.

Die immunmodulatorische Aktivität der hypoallergenen Fragmente, die Polypeptiden mit T-Zell-Epitopen entsprechen, sowie die der hypoallergenen Punktmutanten (z.B. Cystein-Austausche) ist durch ihre Reaktion mit T-Zellen von Graspollenallergikern nachgewiesen.

Solche hypoallergenen Fragmente bzw. Punktmutanten der Cysteine können als Präparate zur Hyposensibilisierung von Allergikern eingesetzt werden, da sie mit gleicher Effektivität mit den T-Zellen reagieren, jedoch aufgrund der verminderten oder ganz fehlenden IgE-Reaktivität zu geringeren IgE-vermittelten Nebenwirkungen führen.

Werden die für die hypoallergenen Phl p 4-Varianten kodierenden Nukleinsäuren oder die unveränderte für Phl p 4 kodierende DNA mit einem humanen Expressionsvektor ligiert, können diese Konstrukte ebenfalls als Präparate für eine Immuntherapie (DNA-Vakzinierung) angewendet werden.

Eine weitere Gruppe von erfindungsgemäßen pharmazeutischen Zubereitungen enthält anstelle der DNA mindestens ein zuvor beschriebenes Polypeptid und eignet sich zur Diagnose und/oder Behandlung besagter Allergien.

Pharmazeutische Zubereitungen im Sinne der vorliegenden Erfindung enthalten als Wirkstoffe ein erfindungsgemäßes Polypeptid oder einen Expressionsvektor und/oder deren jeweilige pharmazeutisch verwendbaren Derivate, einschließlich deren Mischungen in allen Verhältnissen. Hierbei können die erfindungsgemäßen Wirkstoffe zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.
Als Hilfsstoffe sind immunstimulierende DNA oder Oligonukleotide mit CpG-Motiven besonders geeignet.

Diese Zubereitungen können als Therapeutika oder Diagnostika in der Human- oder Veterinärmedizin verwendet werden. Als Trägerstoffe kommen organische oder anorganische Substanzen in Frage, die sich für die parenterale Applikation eignen und die Wirkung des erfindungsgemäßen Wirkstoffs nicht negativ beeinflussen. Zur parenteralen Anwendung dienen insbesondere Lösungen, vorzugsweise ölige oder wässrige Lösungen, ferner Suspensionen, Emulsionen oder Implantate. Der erfindungsgemäße Wirkstoff kann auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen und/oder mehrere weitere Wirkstoffe enthalten.
Weiterhin können durch entsprechende Formulierung des erfindungsgemäßen Wirkstoffs Depotpräparate erhalten werden.

Die Erfindung dient somit auch zur Verbesserung der *in vitro* Diagnostik im Rahmen einer Allergen-Komponenten auflösenden Identifizierung des patientenspezifischen Sensibilisierungsspektrums. Die Erfindung dient ebenfalls zur Herstellung von deutlich verbesserten Präparaten zur spezifischen Immuntherapie von Gräserpollenallergien.

**Tabelle 1 Aminosäuresequenz von Phl p 4-Peptiden**

| **Präparat** | **Peptid Charge** | **SEQ ID NO** | **Aminosäuren** | | | | | | |
|---|---|---|---|---|---|---|---|---|---|
| | | | **1** | **6** | **11** | **16** | **21** | **26** | **31** |
| Intaktes Phl p 4 | P1-a | 35 | YFPP'P' | AAKED | FLGXL | VKEIP | PRLLY | AKSSP | AYP |
| | P1-b | 36 | YFPP'P' | AAKED | FLGXL | VKE-P | PRLLY | AKSSP | |
| | P1-c | 37 | YFPXX | AAKED | FLGXL | | | | |
| | P1-d | 38 | YFPXX | AKKED | FLGXL | | | | |
| | P1-e | 39 | YFPXX | AAKDD | FLGXL | | | | |
| | P1-f | 40 | YFPXX | LANED | F | | | | |
| Glu-C-Fragmente | P2 | 41 | SATPF | XHRKG VLFNI QYV | | | | | |
| | P3 | 42 | GLXYR | XLXPE | | | | | |
| Lys-C-Fragmente | P4 | 43 | KXMGD | DHFXA | VR | | | | |
| | P5 | 44 | APEGA | VDI I | | | | | |
| CNBr-Fragment | P6 | 45 | MEPYV | SINPV | QAYAN Y | | | | |

**Tabelle 2 Degenerierte und spezifische Sense- und Anti-Sense-Primer, die auf der Grundlage von Phl p 4 Peptidsequenzen und DNA-Sequenzen konstruiert wurden**

| **Primer Nr.** | **Peptid/ DNA** | **Sense/ Anti-Sense** | **SEQ ID NO** | **Nukleotidsequenz** |
|---|---|---|---|---|
| 29 | Phl p 4-P1 | s | 46 | |
| 30 | Phl p 4-P2 | s | 47 | |
| 37 | Phl p 4-P6 | as | 48 | |
| 82 | Phl p 4-DNA-NYW | s | 49 | |
| 85 | Phl p 4-DNA-GLV | as | 50 | |
| 86 | Phl p 4-DNA-QRL | as | 51 | |
| 88 | Phl p 4-DNA-PSV | s | 52 | |

Die Nukleotidsequenzen der Primer 82, 85, 86 und 88 ist in dem üblichen 4-Buchstaben-Code dargestellt. Bei den Primern 29, 30 und 37 wird der IUPAC-IUB-DNA-Code verwendet; der Buchstabe 'N' steht hier für Inosin.

**Tabelle 3 Detektierte Einzelnukleotidaustausche**

| Position in Sequenz | Nukleotid gemäß SEQ ID NO 1 | Detektierte SNPs |
|---|---|---|
| 85 | T | A |
| 130 | C | A |
| 159 | G | A |
| 160 | A | C |
| 169 | G | A |
| 185 | C | T |
| 186 | C | A |
| 222 | G | C |
| 226 | G | A |
| 227 | G | C |
| 228 | T | C |
| 237 | C | T |
| 273 | C | T |
| 285 | C | T |
| 286 | C | T |
| 298 | G | A |
| 299 | A | C |
| 303 | C | T |
| 309 | C | G |
| 318 | T | C |
| 320 | G | A |
| 333 | C | G |
| 348 | G | C |
| 369 | C | G |
| 409 | C | T |
| 411 | C | T |
| 420 | T | C |
| 421 | A | C |
| 423 | A | C |
| 424 | G | A |
| 425 | T | C |
| 456 | C | G |
| 462 | C | A |
| 522 | G | C |
| 525 | C | G |
| 567 | G | A |
| 618 | C | T |
| 655 | A | C |
| 657 | G | A |
| 662 | G | A |
| 680 | C | T |
| 684 | G | C |
| 690 | C | A |
| 691 | G | A |
| 693 | G | A |
| 703 | C | T, A |
| 710 | A | C |
| 711 | G | A |
| 713 | C | T |
| 743 | G | A |
| 750 | G | A |
| 768 | C | T |
| 773 | A | C |
| 790 | G | A |
| 798 | G | C |
| 801 | G | A |
| 804 | C | G |
| 809 | C | A |
| 834 | G | C |
| 844 | C | A |
| 859 | A | T |
| 865 | A | G |
| 879 | G | C |
| 895 | G | C |
| 900 | G | C, A |
| 918 | G | A |
| 961 | A | G |
| 962 | A | C |
| 964 | A | C |
| 987 | G | C |
| 994 | A | T |
| 1020 | G | A |
| 1023 | G | C |
| 1036 | G | C |
| 1040 | C | T |
| 1041 | G | C |
| 1047 | C | A |
| 1051 | A | G |
| 1052 | G | A, C |
| 1053 | G | A, C, T |
| 1056 | G | C |
| 1069 | T | C |
| 1073 | G | A |
| 1084 | C | G |
| 1086 | G | C |
| 1090 | C | T |
| 1098 | G | C |
| 1151 | G | C |
| 1152 | G | C |
| 1155 | G | C |
| 1161 | G | C |
| 1185 | C | G |
| 1229 | G | C |
| 1233 | G | C |
| 1239 | A | C |
| 1240 | T | C |
| 1242 | G | C |
| 1257 | G | C |
| 1266 | C | T |
| 1269 | C | T |
| 1278 | A | C, G |
| 1305 | C | G |
| 1308 | C | T |
| 1311 | C | A |
| 1335 | G | C |
| 1350 | G | C |
| 1357 | T | A |
| 1359 | A | G |
| 1370 | G | C |
| 1377 | T | C |
| 1378 | T | A |
| 1379 | T | A |
| 1383 | G | C |
| 1398 | C | T |
| 1411 | T | C |
| 1414 | C | G |
| 1425 | C | A |
| 1428 | C | T |
| 1443 | G | C |
| 1449 | C | T |
| 1464 | G | A |
| 1485 | G | A |
| 1498 | A | C |

**Tabelle 4 Aminosäureaustausche, infolge von Einzelnukleotidaustauschen**

| Position in Sequenz | Aminosäure gemäß SEQ ID NO 2 | Detektierte Austausche |
|---|---|---|
| 6 | A | L |
| 7 | A | K |
| 8 | K | N |
| 9 | E | D |
| 29 | S | T |
| 54 | I | L |
| 57 | V | I |
| 62 | A | V |
| 76 | G | T, N, S |
| 100 | E | T |
| 107 | S | N |
| 137 | H | Y |
| 141 | T | P |
| 142 | V | A, T |
| 189 | T | K |
| 219 | K | Q |
| 221 | R | K |
| 227 | P | L |
| 231 | V | I |
| 235 | P | T, S |
| 237 | K | T |
| 238 | A | V |
| 248 | R | K |
| 258 | D | A |
| 264 | V | I |
| 270 | T | K |
| 282 | Q | K |
| 287 | M | L |
| 289 | S | G |
| 299 | A | P |
| 321 | N | A |
| 322 | I | L |
| 332 | T | S |
| 346 | E | Q |
| 347 | P | L |
| 351 | R | E, T |
| 357 | F | L |
| 358 | S | N |
| 362 | L | V |
| 364 | P | S |
| 384 | W | S |
| 410 | G | A |
| 419 | E | D |
| 456 | F | Y |
| 457 | S | A, N |
| 460 | L | K |
| 468 | K | M |
| 472 | Q | E |
| 498 | K | Q |

**Tabelle 5 Abweichende Aminosäurepositionen bei einzelnen rekombinanten Phl p 4-Klonen gegenüber SEQ ID NO 2**

| **Beispiel** | **Abweichende Positionen*** |
|---|---|
| Klon 1 | L54, I57, V62, S76, T100, N107, Y137, P141, T142, K189, Q219, K221, L227, I231, S235, T237, V238, K248, A258, I264, K270, K282, L287, P299, A321, L322, S332, Q346, P347, T351, L357, N358, V362, S384, A410, D419, Y456, A457, K460, E472 |
| Klon 2 | L54, I57, V62, T76, T100, N107, Y137, P141, T142, K189, Q219, K221, I231, S235, T237, V238, K248, A258, I264, K270, K282, L287, P299, A321, L322, S332, Q346, P347, T351, L357, N358, V362, S384, A410, D419, Y456, A457, K460, E472 |
| Klon 3 | P141, K282, L287, P299, L347, E351 |
| Klon 4 | G289, A410, D419, Y456, A457, K460, E472 |
| Klon 5 | L347, E351, S384, A410, D419, Y456, A457, K460, E472 |
| Klon 6 | N107, Y137, P141, T142, K189, Q219, K221, I231, S235, T237, V238, K248, A258, I264, K270, K282, L287, P299, A321, L322, S332, Q346, P347, T351, L357, N358, V362, S384, A410, D419, Y456, A457, K460 |
| Klon 7 | K248, A258, I264, K270, K282, L287, P299, A321, L322, S332, Q346, P347, T351, L357, N358, V362, S384 |
| Klon 8 | Q219, K221, I231, S235, T237, V238, K248, A258, I264, K270, K282, L287, P299, E351 |
| Klon 9 | M231, T246, A251, C263, G289, L307, L309, E334 |
| Klon 10 | Q219, K221, I231, S235, T237, M238, V242, V246, K248, A258, I264, K270, K282, L287, P299, A321, L322, S332, Q346, P347, T351, N358, V362, S384, Insertion GA zwischen Position 407 und 408, N452, Y456, A457, K460, E472 |
| Klon 11 | Insertion GA zwischen Position 407 und 408 |

| | |
|---|---|
| *[Aminosäure gemäß SEQ ID NO 2 / Position in Sequenz / Abweichende Aminosäure] | |

**Tabelle 6 Aminosäurezusammensetzung des Phl p 4**

| **Aminosäuren** | **Anzahl** | **Gewichts-%** |
|---|---|---|
| Geladene | 138/138/138 | 33,89/33,86/33,93 |
| Säure | 45/46/43 | 9,82/10,05/9,38 |
| Basische | 54/53/55 | 13,67/13,39/13,78 |
| Polare | 120/119/124 | 24,88/24,71/25,89 |
| Hydrophobe | 180/180/180 | 35,64/35,66/35,43 |
| A Ala | 40/40/41 | 5,10/5,10/5,24 |
| C Cys | 5/5/5 | 0,92/0,93/0,93 |
| D Asp | 24/24/24 | 4,95/4,96/4,97 |
| E Glu | 21/22/19 | 4,86/5,10/4,41 |
| F Phe | 24/24/22 | 6,33/6,34/5,82 |
| G Gly | 42/42/40 | 4,30/4,30/4,10 |
| H His | 10/10/9 | 2,46/2,46/2,22 |
| I Ile | 29/29/30 | 5,88/5,89/6,10 |
| K Lys | 29/29/33 | 6,67/6,67/7,60 |
| L Leu | 33/33/35 | 6,70/6,70/7,12 |
| M Met | 11/11/10 | 2,59/2,59/2,36 |
| N Asn | 22/22/23 | 4,50/4,50/4,72 |
| P Pro* | 38/39/39 | 6,62/6,80/6,81 |
| Q Gln | 15/15/15 | 3,45/3,45/3,46 |
| R Arq | 25/24/22 | 7,00/6,73/6,18 |
| S Ser | 32/32/33 | 5,00/5,00/5,17 |
| TThr | 22/21/22 | 3,99/3,81/4,00 |
| V Val | 41/41/40 | 7,29/7,29/7,13 |
| W Trp | 13/13/12 | 4,34/4,34/4,02 |
| Y Tyr | 24/24/26 | 7,02/7,03/7,63 |

| | | |
|---|---|---|
| * einschließlich Hydroxyprolin | | |

Die Werte sind für die drei dominierenden Sequenzen in der Reihenfolge SEQ ID NO 2 / SEQ ID NO 4 / SEQ ID NO 6 angegeben.

## Patentansprüche

1. Ein DNA-Molekül mit der Nukleotidsequenz SEQ ID NO 1 mit einem Einzelnukleotidaustausch ausgewählt aus der Gruppe bestehend aus T85A, C130A, G159A, A160C, G169A, G222C, G226A, G227C, T228C, C237T, C285T, C286T, G298A, A299C, C303T, C309G, T318C, G320A, C333G, G348C, C369G, C409T, C411T, T420C, A421C, A423C, T425C, G522C, C525G, C618T, G657A, G662A, G684C, C690A, G691A, G693A, C703A, A710C, G711A, G743A, G750A, C768T, G798C, G801A, C804G, G834C, A865G, G879C, G918A, A994T, C1047A, A1051G, G1052A, G1053A, C1090T, G1098C, C1185G, A1278C, G1370C, C1414G, C1425A, C1428T, G1443C, C1449T und G1464A.

2. Ein DNA-Molekül, kodierend für ein Polypeptid mit der Aminosäuresequenz SEQ ID NO 2 mit einem Aminosäureaustausch ausgewählt aus der Gruppe bestehend aus A6L, A7K, K8N, E9D, S29T, I54L, V57I, G76T, E100T, S107N, H137Y, T141P, V142A, R221K, V231I, P235T, K237T, R248K, S289G, T332S, R351E, P364S, Q472E und K498Q.

3. Ein DNA-Molekül, entsprechend einer Nukleotidsequenz gemäß der Ansprüche 1 oder 2, kodierend für ein immunmodulatorisches T-Zell reaktives Fragment, **dadurch gekennzeichnet, dass** besagte Nukleotidsequenz durch gezielten Austausch eines, mehrerer oder aller Cysteine des entsprechenden Polypeptids gegen eine andere Aminosäure gezielt verändert wurde.

4. Ein rekombinanter DNA-Expressionsvektor, enthaltend ein DNA-Molekül gemäß der Ansprüche 1 bis 3, funktionell verbunden mit einer Expressionskontrollsequenz.

5. Ein Wirtsorganismus, transformiert mit einem DNA-Molekül gemäß der Ansprüche 1 bis 3 oder einem Expressionsvektor gemäß Anspruch 4.

## Claims

1. A DNA molecule having the nucleotide sequence SEQ ID NO 1 with a single nucleotide polymorphism selected from the group consisting of T85A, C130A, G159A, A160C, G169A, G222C, G226A, G227C, T228C, C237T, C285T, C286T, G298A, A299C, C303T, C309G, T318C, G320A, C333G, G348C, C369G, C409T, C411T, T420C, A421C, A423C, T425C, G522C, C525G, C618T, G657A, G662A, G684C, C690A, G691A, G693A, C703A, A710C, G711A, G743A, G750A, C768T, G798C, G801A, C804G, G834C, A865G, G879C, G918A, A994T, C1047A, A1051G, G1052A, G1053A, C1090T, G1098C, C1185G, A1278C, G1370C, C1414G, C1425A, C1428T, G1443C, C1449T and G1464A.

2. A DNA molecule encoding for a polypeptide having the amino acid sequence SEQ ID NO 2 with an amino acid polymorphism selected from the group consisting of A6L, A7K, K8N, E9D, S29T, I54L, V57I, G76T, E100T, S107N, H137Y, T141P, V142A, R221K, V231I, P235T, K237T, R248K, S289G, T332S, R351E, P364S, Q472E and K498Q.

3. A DNA molecule corresponding to a nucleotide sequence according to Claim 1 or 2, encoding for an immunomodulatory T-cell-reactive fragment, **characterised in that** said nucleotide sequence has been specifically modified by specific replacement of one, several or all cysteines of the corresponding polypeptide by another amino acid.

4. A recombinant DNA expression vector containing a DNA molecule according to Claims 1 to 3, functionally connected to an expression control sequence.

5. A host organism transformed with a DNA molecule according to Claims 1 to 3 or an expression vector according to Claim 4.

## Revendications

1. Molécule d'ADN comportant la séquence de nucléotides SEQ ID NO 1 avec un polymorphisme de nucléotide unique qui est sélectionné parmi le groupe qui est constitué par T85A, C130A, G159A, A160C, G169A, G222C, G226A, G227C, T228C, C237T, C285T, C286T, G298A, A299C, C303T, C309G, T318C, G320A, C333G, G348C, C369G, C409T, C411T, T420C, A421C, A423C, T425C, G522C, C525G, C618T, G657A, G662A, G684C, C690A, G691A, G693A, C703A, A710C, G711A, G743A, G750A, C768T, G798C, G801A, C804G, G834C, A865G, G879C, G918A, A994T, C1047A, A1051G, G1052A, G1053A, C1090T, G1098C, C1185G, A1278C, G1370C, C1414G, C1425A, C1428T, G1443C, C1449T et G1464A.

2. Molécule d'ADN codant pour un polypeptide comportant la séquence d'acides aminés SEQ ID NO 2 avec un polymorphisme d'acides aminés qui est sélectionné parmi le groupe qui est constitué par A6L, A7K, K8N, E9D, S29T, I54L, V57I, G76T, E100T, S107N, H137Y, T141P, V142A, R221K, V231I, P235T, K237T, R248K, S289G, T332S, R351E, P364S, Q472E et K498Q.

3. Molécule d'ADN correspondant à une séquence de nucléotides selon la revendication 1 ou 2, codant pour un fragment immunomodulateur réactif aux cellules T, **caractérisée en ce que** ladite séquence de nucléotides a été modifiée spécifiquement au moyen du remplacement spécifique d'une cystéine, de plusieurs cystéines ou de toutes les cystéines du polypeptide correspondant par un autre acide aminé.

4. Vecteur d'expression d'ADN recombinant contenant une molécule d'ADN selon les revendications 1 à 3, fonctionnellement en connexion avec une séquence de contrôle d'expression.

5. Organisme hôte transformé avec une molécule d'ADN selon les revendications 1 à 3 ou un vecteur d'expression selon la revendication 4.
